# EUROPEAN PATENT APPLICATION

(11) **EP 4 852 006 A1**
(43) Date of publication of application: **09.12.2026**
(21) Application number: 26177819.5
(22) Date of filing: 11.05.2026
(51) Int. Cl.: G16H 10/60, G16H 15/00

(54) **METHODS AND SYSTEMS FOR AUTOMATIC DIGITIZATION OF MEDICAL PROTOCOLS**

(30) Priority: 03.06.2025 US 202519227152
(71) Applicant: GE Precision Healthcare LLC, Waukesha, WI 53188 (US)
(72) Inventor: TOMAR, Tarun, 560066 Bengaluru (IN); SHERIKAR, Vinod, 560066 Bengaluru (IN); GUPTA, Vivek, 560066 Bengaluru (IN); SINGH, Ranvijay, 560066 Bengaluru (IN)
(74) Representative: Kilburn & Strode LLP

(57) **Abstract**

A method (200) for digitizing medical protocols is described. The method (200) includes receiving an unstructured medical protocol (114), converting the unstructured medical protocol (114) into machine-readable text, analyzing the machine-readable text using natural language processing to identify action nodes representing medical actions, identify decision nodes representing conditional branches, and determine relationships between the action nodes and decision nodes. The method (200) further includes generating a protocol graph structure (318) based on the identified nodes and relationships, wherein the protocol graph structure (318) comprises nodes and edges, each node includes a position identifier and a parent position identifier, and the edges represent transitions between nodes. The method (200) also includes storing the protocol graph structure (318) in a database (112) and generating a visual representation of the protocol graph structure (318) showing available paths through the medical protocol.

## Description

### TECHNICAL FIELD

Embodiments of the subject matter disclosed herein relate to healthcare information systems, and more particularly, to methods and systems for automatic digitization and management of medical protocols.

### BACKGROUND

In In the healthcare industry, medical protocols and guidelines are integral for ensuring consistent, high-quality patient care. These protocols provide standardized approaches for diagnosing and treating various medical conditions, performing procedures, and managing patient care. Traditionally, these protocols have been maintained in paper formats or as disparate digital documents, leading to challenges in accessibility, version control, and integration with modern electronic healthcare systems.

The manual management of medical protocols can result in inefficiencies, delays in updates, and potential discrepancies in patient care. Healthcare providers often need to locate physical copies of protocols or search through multiple digital repositories, which can be time-consuming and may lead to the use of outdated information. Additionally, paper-based protocols cannot be easily integrated with hospital information systems (HIS), electronic health records (EHR), or other digital healthcare platforms that have become standard in modern healthcare facilities.

Current approaches to digitizing medical protocols typically involve manual configuration of each protocol step within healthcare applications. This process is labor-intensive, time-consuming, and feasible only for pre-configured protocols. There is no efficient means of converting handwritten or plain text protocols into digital formats that can dynamically guide healthcare providers through appropriate care pathways based on patient conditions and clinical decisions.

Furthermore, existing systems lack the ability to automatically track protocol adherence, document the steps performed, and integrate with patient data in real-time. This limitation makes it difficult to ensure compliance with established care standards, conduct quality audits, and analyze protocol effectiveness across patient populations.

The absence of standardized digital protocol management also creates challenges for healthcare facilities in maintaining regulatory compliance, as there is limited ability to document that proper procedures were followed according to established guidelines. Additionally, the lack of integration between protocols and patient data means that healthcare providers must manually determine which protocols are appropriate based on patient conditions, rather than having the system suggest relevant protocols automatically.

Therefore, there remains an unmet need for an automated system that can efficiently convert various forms of medical protocols into interactive digital formats, integrate these protocols with hospital information systems, track protocol adherence, and provide dynamic guidance to healthcare providers based on patient-specific data. Such a system would improve workflow efficiency, enhance patient care quality, reduce the risk of human error, and support better coordination among healthcare providers.

### SUMMARY

In one example, a method for digitizing medical protocols includes receiving an unstructured medical protocol, converting the unstructured medical protocol into machine-readable text, analyzing the machine-readable text using natural language processing to identify action nodes representing medical actions, identify decision nodes representing conditional branches, and determine relationships between the action nodes and decision nodes. The method further includes generating a protocol graph structure based on the identified nodes and relationships, wherein the protocol graph structure comprises nodes and edges, each node includes a position identifier and a parent position identifier, and the edges represent transitions between nodes. The method also includes storing the protocol graph structure in a database and generating a visual representation of the protocol graph structure showing available paths through the medical protocol. This method addresses the challenges of manual protocol management by providing an automated system for converting various forms of medical protocols into interactive digital formats that can be easily accessed, updated, and integrated with electronic healthcare systems.

In another example, a system for digitizing medical protocols includes a computing device comprising one or more processors, a display device, and a memory storing instructions executable by the one or more processors. The instructions are executable to receive an unstructured medical protocol via an input interface, wherein the unstructured medical protocol comprises at least one of handwritten text, plain text, or spoken input, convert the unstructured medical protocol into machine-readable text, and analyze the machine-readable text using natural language processing to identify action nodes representing medical actions to be performed, identify decision nodes representing patient state-dependent branches, and determine relationships between the action nodes and decision nodes. The instructions are further executable to generate a protocol graph structure comprising nodes representing the identified action nodes and decision nodes, wherein each node includes a position identifier and a parent position identifier, and edges representing the determined relationships between nodes, receive patient state data from a hospital information system, automatically update a current position within the protocol graph structure based on the received patient state data, and output, to the display device, a visual representation of the protocol graph structure showing available paths through the medical protocol, the current position within the protocol graph structure, and historical progression through previously traversed nodes. This system enhances workflow efficiency by providing healthcare providers with dynamic guidance based on patient-specific data and enabling real-time protocol adherence tracking.

In yet another example, a method for digitizing and managing medical protocols in a hospital information system (HIS) includes preprocessing an unstructured medical protocol by receiving the unstructured medical protocol in at least one of handwritten, spoken, or plain text format, converting the unstructured medical protocol into machine-readable text, and sanitizing the machine-readable text by removing extraneous characters and standardizing medical terminology. The method further includes analyzing the preprocessed text using a trained natural language processing (NLP) model to identify action nodes representing specific medical procedures using named entity recognition, identify decision nodes representing patient state-dependent branches by detecting conditional statements and threshold values, determine temporal and causal relationships between the identified nodes based on textual indicators and sentence structure, and extract measurement thresholds and clinical parameters associated with the decision nodes. The method also includes generating a protocol graph structure, creating a protocol instance for a specific patient, and displaying, on a graphical user interface, a visual representation of the protocol graph structure, the current position within the protocol graph structure, available paths forward based on current patient state data, and historical progression through previously traversed nodes. This comprehensive method improves patient care quality by ensuring consistent application of medical protocols while maintaining detailed documentation for regulatory compliance and quality assurance purposes.

The above advantages and other advantages, and features of the present description will be readily apparent from the following Detailed Description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

Various aspects of this disclosure may be better understood upon reading the following detailed description and upon reference to the drawings in which:
FIG. 1 is a block diagram of a medical protocol digitization system in accordance with an embodiment of the present invention.
FIG. 2 is a flowchart illustrating a method for digitizing medical protocols in accordance with an embodiment of the present invention.
FIG. 3 is a block diagram of a natural language processing pipeline for analyzing preprocessed medical protocol text in accordance with an embodiment of the present invention.
FIG. 4 is a flowchart illustrating a method for generating and validating a protocol graph structure in accordance with an embodiment of the present invention.
FIG. 5 is a flowchart illustrating a method for integrating with a hospital information system in accordance with an embodiment of the present invention.
FIG. 6 is a flowchart illustrating a method for creating and managing a protocol instance for a specific patient in accordance with an embodiment of the present invention.
FIG. 7 is a flowchart illustrating a method for managing related protocols and protocol updates in accordance with an embodiment of the present invention.
FIG. 8 is a flowchart illustrating a method for version control and audit trail management of protocol modifications in accordance with an embodiment of the present invention.
FIG. 9 is a flowchart illustrating a method for managing patient state and protocol position updates in accordance with an embodiment of the present invention.
FIG. 10 is a flowchart illustrating a method for updating a user interface based on protocol state changes in accordance with an embodiment of the present invention.

### DETAILED DESCRIPTION

The following description relates to methods and systems for automatic digitization of medical protocols. In healthcare settings, medical protocols and guidelines provide standardized approaches for diagnosing and treating various medical conditions, performing procedures, and managing patient care. These protocols have traditionally been maintained in paper formats or as disparate digital documents, creating challenges in accessibility, version control, and integration with electronic healthcare systems. Converting these unstructured protocols into structured digital formats presents significant technical challenges, including accurate extraction of decision logic, establishment of proper node relationships, and integration with existing hospital information systems.

The systems and methods described herein address these technical challenges by providing an automated approach to digitizing medical protocols and integrating them with hospital information systems. By employing natural language processing techniques, the system can identify action nodes, decision nodes, and their relationships within unstructured text, and convert them into a structured protocol graph that can be stored, visualized, and integrated with patient data. This improves the computational efficiency of protocol management, reduces storage requirements compared to maintaining multiple versions of documents, and enables real-time data processing for protocol adherence.

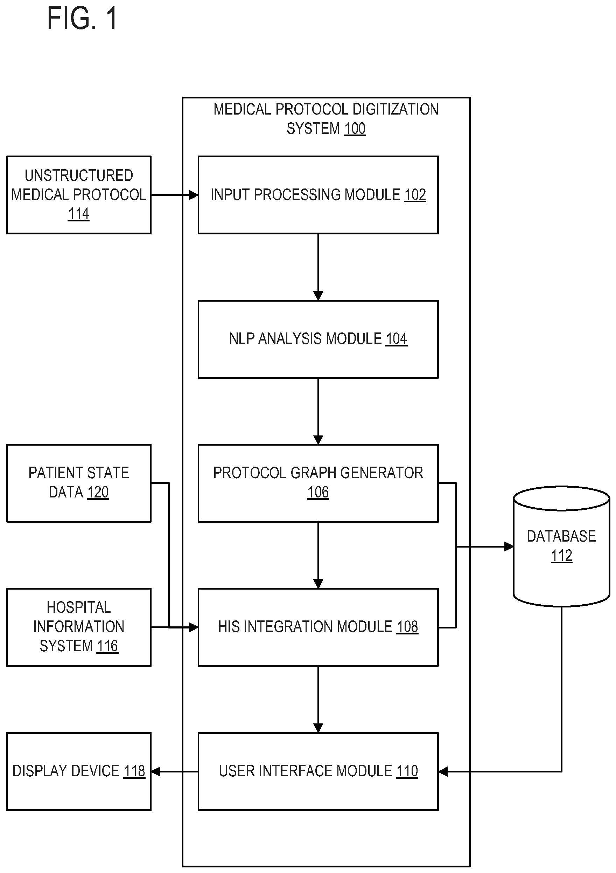

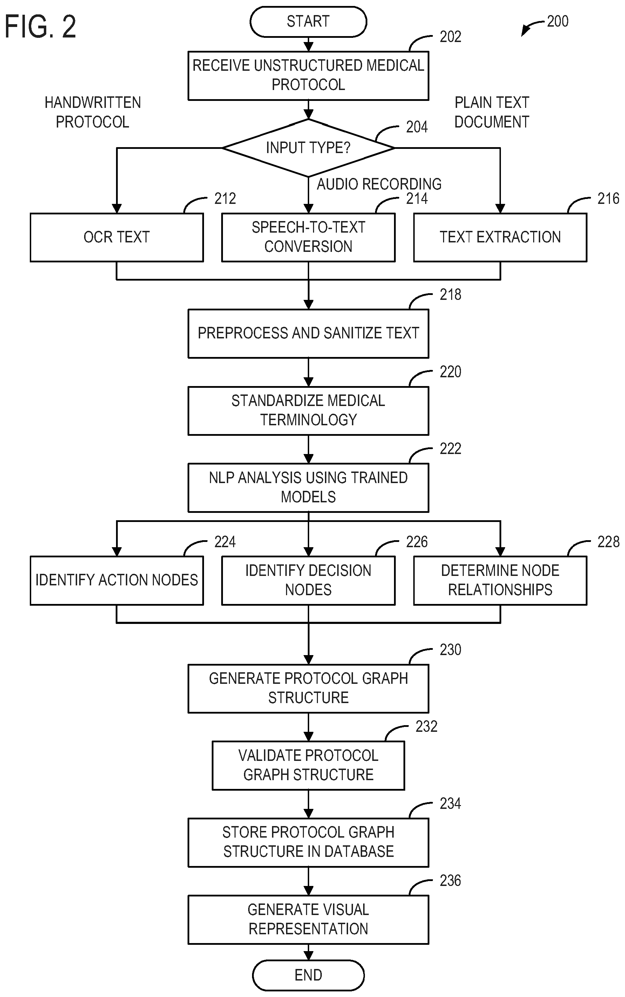

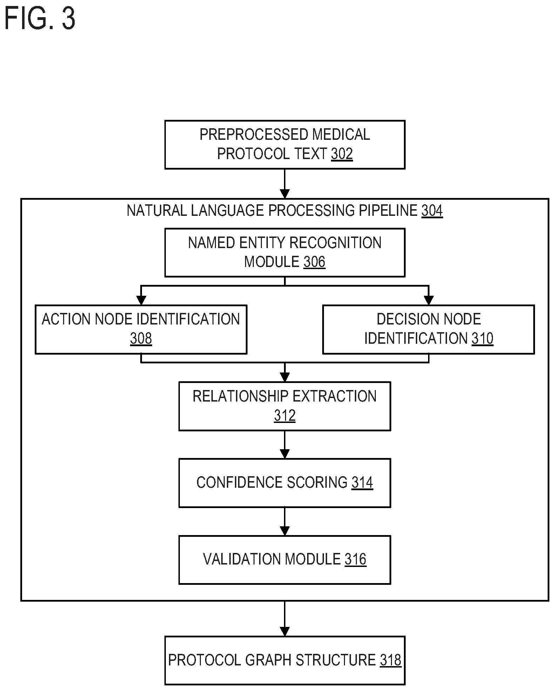

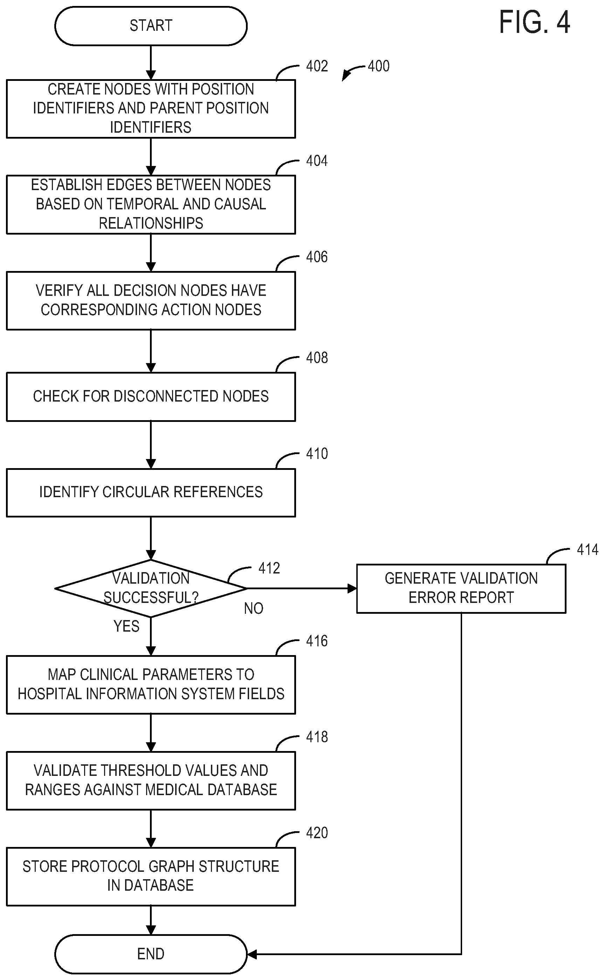

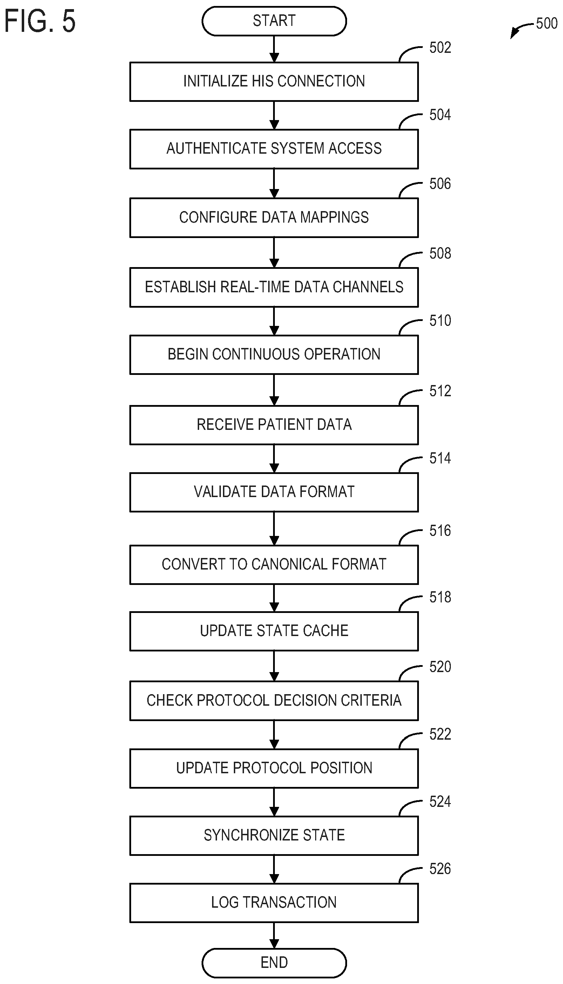

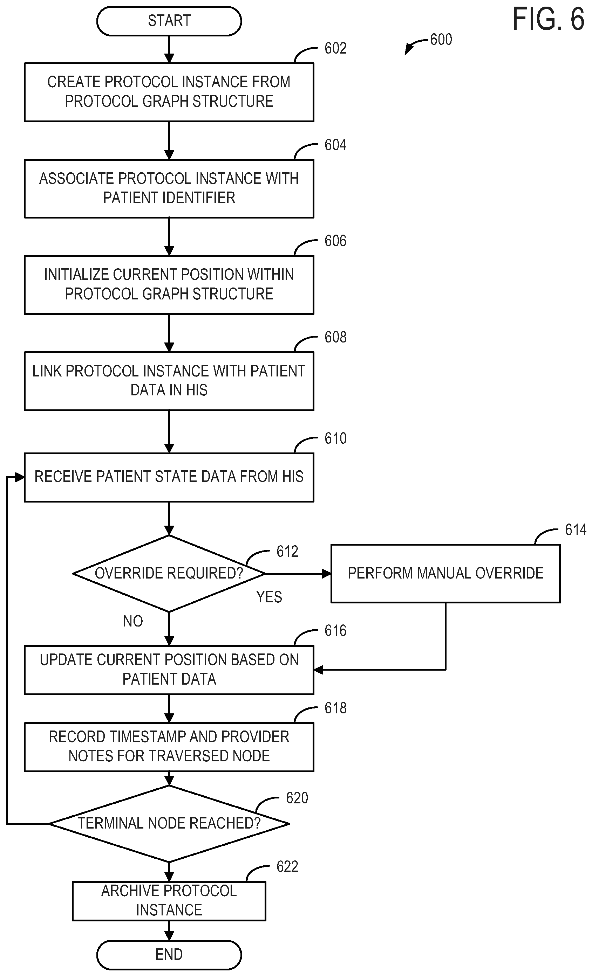

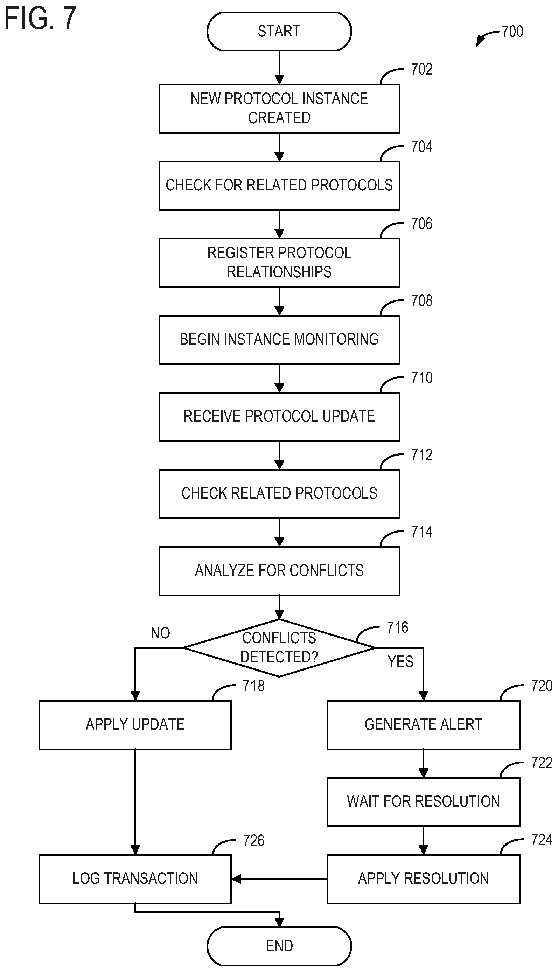

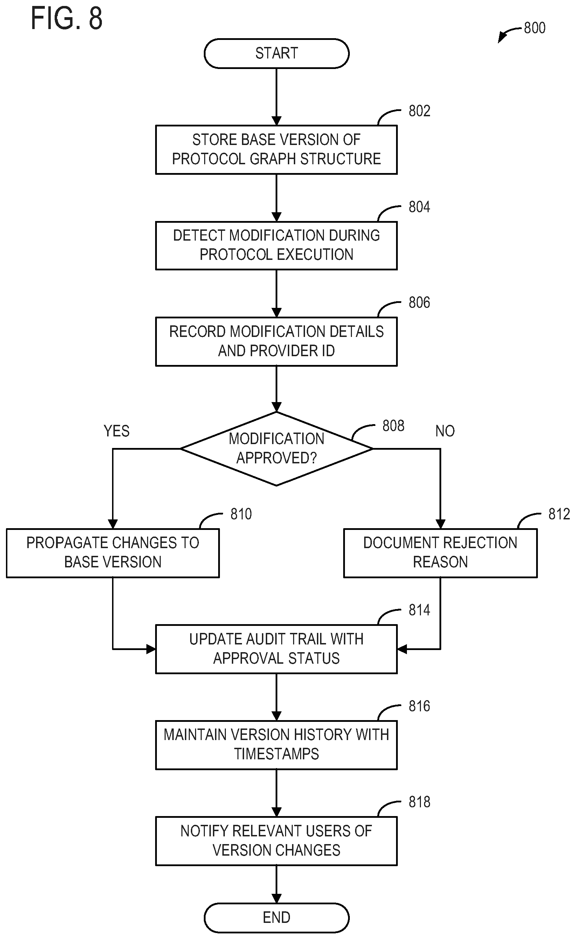

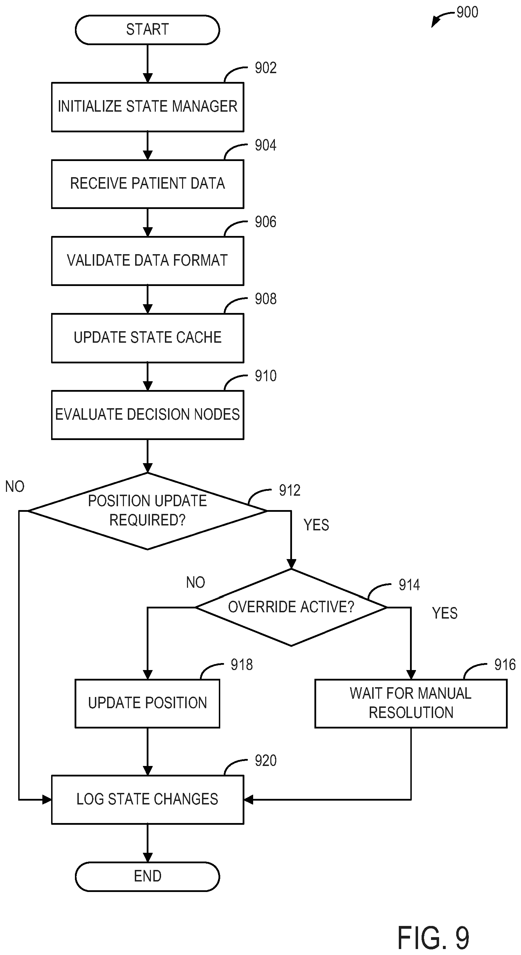

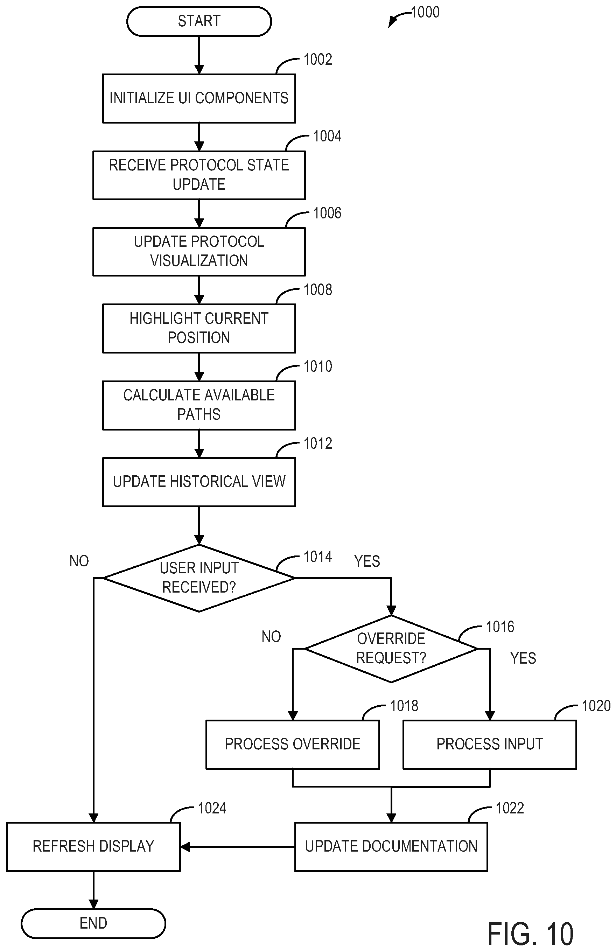

The figures illustrate various aspects of a medical protocol digitization system that transforms unstructured protocols into interactive digital formats. FIG. 1 shows a block diagram of a medical protocol digitization system, illustrating components that process input protocols, analyze them using natural language processing, generate protocol graph structures, and integrate with hospital information systems. FIG. 2 presents a flowchart of a digitization method, detailing the process from receiving unstructured protocols through preprocessing, analysis, and graph generation. FIG. 3 expands on a natural language processing pipeline that processes the system's data, showing how preprocessed text is analyzed to extract structured information. FIG. 4 illustrates a process of generating and validating a protocol graph structure, including node creation, edge establishment, and validation procedures. FIG. 5 details integration mechanisms with hospital information systems, showing how the system establishes connections, configures data mappings, and processes patient data. FIGS. 6-10 illustrate various technical aspects of the system's operation, including protocol instance management (FIG. 6), related protocol handling (FIG. 7), version control mechanisms (FIG. 8), patient state management (FIG. 9), and user interface updates (FIG. 10).

The systems and methods described herein provide technical advantages over existing approaches to medical protocol management. The automated conversion of unstructured protocols into structured digital formats reduces computational overhead associated with document management and enables more efficient data storage and retrieval. The graph-based representation of protocols allows for optimized traversal algorithms when determining appropriate care paths based on patient data. The integration with hospital information systems creates a framework for automated data exchange, reducing manual data entry and the associated error rates. The system's ability to process real-time patient data and automatically update protocol positions demonstrates a technical improvement in data processing efficiency compared to manual protocol management.

Additionally, the structured digital representation enables computational analysis of protocols that would be impossible with traditional document formats. The system can automatically detect conflicts between protocols, identify circular references, and validate the integrity of the protocol graph structure. The version control mechanisms provide technical solutions for tracking changes, maintaining audit trails, and ensuring data consistency across distributed systems. These technical improvements enhance the reliability and performance of healthcare information systems while reducing the computational resources required for protocol management.

Referring to FIG. 1, a medical protocol digitization system 100 for processing and analyzing medical protocols is shown. The system 100 comprises multiple internal processing modules that interface with external data sources and output devices. The medical protocol digitization system 100 is configured to convert unstructured medical protocols into structured digital formats that can be integrated with hospital information systems and used to guide healthcare providers through appropriate care pathways based on patient conditions and clinical decisions.

The unstructured medical protocol 114 represents the input data for the medical protocol digitization system 100. This may include handwritten protocol documents, audio recordings of spoken protocols, or plain text documents containing medical guidelines. The unstructured medical protocol 114 is provided to the input processing module 102, which converts it into a format suitable for analysis. The conversion of these unstructured protocols into structured digital formats addresses the challenges of manual protocol management by providing an automated system that can be easily accessed, updated, and integrated with electronic healthcare systems.

The input processing module 102 is configured to receive and process unstructured medical protocols 114 from various sources. In one embodiment, the input processing module 102 receives handwritten protocol documents and converts them into machine-readable text using optical character recognition (OCR) techniques. In another embodiment, the input processing module 102 processes audio recordings of spoken medical protocols using speech-to-text conversion algorithms. In yet another embodiment, the input processing module 102 extracts text from plain text documents or digital files containing medical protocols. The input processing module 102 also performs preprocessing and sanitization of the text by removing extraneous characters and standardizing medical terminology using a medical terminology dictionary. This preprocessing enhances the accuracy of subsequent analysis by ensuring consistent terminology throughout the protocol text. The input processing module 102 is operatively connected to the NLP analysis module 104 to provide the preprocessed text for further analysis.

The NLP analysis module 104 is configured to analyze the preprocessed text using natural language processing techniques to identify key elements of the medical protocol. In one embodiment, the NLP analysis module 104 employs a named entity recognition model trained on annotated medical protocols to identify medical actions, such as medication administration and patient monitoring tasks. In another embodiment, the NLP analysis module 104 detects decision nodes by identifying conditional statements and threshold values for patient vital signs. In a third embodiment, the NLP analysis module 104 determines temporal and causal relationships between the identified nodes based on textual indicators and sentence structure. The NLP analysis module 104 may also extract measurement thresholds and clinical parameters associated with decision nodes, which are later used for integration with patient data. The NLP analysis module 104 provides the identified action nodes, decision nodes, and their relationships to the protocol graph generator 106 for construction of a protocol graph structure.

The protocol graph generator 106 is configured to generate a structured protocol graph based on the nodes and relationships identified by the NLP analysis module 104. In one embodiment, the protocol graph generator 106 creates nodes with position identifiers and parent position identifiers to establish a hierarchical structure. In another embodiment, the protocol graph generator 106 establishes edges between nodes based on the temporal and causal relationships identified during NLP analysis. The protocol graph generator 106 also validates the protocol graph structure by verifying that all decision nodes have corresponding action nodes, checking for disconnected nodes, and identifying circular references. Additionally, the protocol graph generator 106 maps clinical parameters in decision nodes to corresponding fields in the hospital information system 116. The protocol graph structure generated by the protocol graph generator 106 is stored in the database 112 for later retrieval and use. The structured representation enables computational analysis of protocols that would be impossible with traditional document formats, allowing for automatic detection of conflicts between protocols and validation of the protocol integrity.

The hospital information system 116 manages patient records, clinical data, and other healthcare information. The hospital information system 116 provides patient state data 120 to the HIS integration module 108 and may receive updates from the medical protocol digitization system 100 regarding protocol adherence and documentation. The integration with the hospital information system 116 enables the medical protocol digitization system 100 to access relevant patient information without requiring manual data entry, improving workflow efficiency and reducing the risk of errors. This integration also allows for the automatic suggestion of relevant protocols based on patient conditions, further enhancing the utility of the system.

The HIS integration module 108 is configured to establish and maintain connections with the hospital information system 116 and process patient state data 120. In one embodiment, the HIS integration module 108 initializes connections with the hospital information system 116, authenticates system access, and configures data mappings between the protocol graph structure and the hospital information system fields. In another embodiment, the HIS integration module 108 establishes real-time data channels to receive patient state data 120 from medical devices connected to the hospital information system 116. The HIS integration module 108 validates the format of received patient data, converts it to a canonical format using standardized units for measurements, and updates a state cache with the processed data. The HIS integration module 108 then checks protocol decision criteria against the patient state data 120 and updates the current position within the protocol graph structure accordingly. This integration with the hospital information system 116 enables automated data exchange, reducing manual data entry and associated error rates.

The user interface module 110 is configured to generate and manage the visual representation of the protocol graph structure on a display device 118. In one embodiment, the user interface module 110 displays the current node in a distinct visual style, available next nodes based on current patient state data, and interactive elements for healthcare provider input at decision nodes. In another embodiment, the user interface module 110 maintains a visual history of traversed nodes, allowing healthcare providers to review the historical progression through previously traversed nodes. The user interface module 110 also processes user inputs, including override requests when healthcare providers need to manually navigate the protocol graph. When an override is requested, the user interface module 110 documents the reasons for the override and obtains authorization based on provider credentials. The user interface module 110 receives protocol state updates from the HIS integration module 108 and refreshes the display accordingly, ensuring that healthcare providers always have access to the most current protocol information.

The patient state data 120 includes real-time and historical patient information that is used to determine the appropriate path through the protocol graph structure. This data may include vital signs, laboratory results, medication information, and other clinical parameters relevant to the medical protocol. The patient state data 120 is received by the HIS integration module 108 from the hospital information system 116 and is used to automatically update the current position within the protocol graph structure. By incorporating real-time patient data into the protocol navigation process, the system can provide dynamic guidance to healthcare providers based on the specific conditions of each patient.

The database 112 stores the protocol graph structures generated by the protocol graph generator 106, as well as protocol instances associated with specific patients. In one embodiment, the database 112 maintains version control of protocol graph structures by storing base versions and tracking modifications made during protocol execution. In another embodiment, the database 112 stores timestamps and healthcare provider notes for each traversed node, creating a comprehensive audit trail of protocol adherence. The database 112 provides stored protocol graph structures to the user interface module 110 for visualization and to the HIS integration module 108 for integration with patient data. The structured storage of protocols in the database 112 reduces computational overhead associated with document management and enables more efficient data retrieval compared to traditional document formats.

The display device 118 presents the visual representation of the protocol graph structure to healthcare providers. The display device 118 receives output from the user interface module 110 and shows the current position within the protocol graph structure, available paths forward based on current patient state data, and historical progression through previously traversed nodes. The visual representation on the display device 118 enhances the usability of the medical protocol digitization system 100 by providing clear guidance to healthcare providers and reducing the cognitive load associated with following complex medical protocols. This improves patient care quality by ensuring consistent application of medical protocols while maintaining detailed documentation for regulatory compliance and quality assurance purposes.

The medical protocol digitization system 100 provides technical advantages over existing approaches to medical protocol management. The automated conversion of unstructured protocols into structured digital formats reduces computational overhead associated with document management and enables more efficient data storage and retrieval. The graph-based representation of protocols allows for optimized traversal algorithms when determining appropriate care paths based on patient data. The system's ability to process real-time patient data and automatically update protocol positions demonstrates a technical improvement in data processing efficiency compared to manual protocol management.

Referring to FIG. 2, a flowchart of a method 200 for digitizing medical protocols is shown. The method 200 provides an automated approach for converting unstructured medical protocols into structured digital formats that can be integrated with hospital information systems. The method 200 begins at operation 202 where an unstructured medical protocol is received.

At operation 202, the method includes receiving an unstructured medical protocol. In one embodiment, the unstructured medical protocol may be received via an input interface of a computing device. The unstructured medical protocol may be in various formats, such as handwritten text on paper documents, audio recordings of spoken protocols, or plain text documents containing medical guidelines. For example, the unstructured medical protocol may include standardized care approaches for diagnosing and treating various medical conditions, performing procedures such as CPR (cardiopulmonary resuscitation), or managing patient care for conditions like anaphylaxis. In another embodiment, the unstructured medical protocol may be received from a hospital database containing legacy protocols that have not yet been digitized or from healthcare providers who have developed custom protocols for specific patient care scenarios.

After receiving the unstructured medical protocol, the method 200 proceeds to operation 204 where the input type of the unstructured medical protocol is determined. In one embodiment, the input type is determined by analyzing file format metadata or by examining visual characteristics of the input. For example, image-based files may indicate handwritten protocols, audio files may indicate spoken protocols, and text-based files may indicate plain text documents. In another embodiment, the input type may be explicitly specified by a user through a selection interface that allows the user to designate the type of protocol being submitted for digitization.

Based on the determined input type at operation 204, the method 200 proceeds to one of three different conversion operations: operation 212 for handwritten protocols, operation 214 for audio recordings, or operation 216 for plain text documents. Only one of these operations is performed based on the input type determined at operation 204.

At operation 212, if the input type is determined to be a handwritten protocol, optical character recognition (OCR) text conversion is performed. In one embodiment, the OCR text conversion utilizes the Tesseract OCR engine to process images of handwritten medical protocols and convert them into machine-readable text. The OCR process analyzes the visual patterns of handwritten characters and maps them to corresponding digital text characters. In another embodiment, the OCR text conversion may include pre-processing steps such as image enhancement, noise reduction, and skew correction to improve recognition accuracy, particularly for medical protocols that may have been handwritten under time constraints in clinical settings.

At operation 214, if the input type is determined to be an audio recording, speech-to-text conversion is performed. In one embodiment, the speech-to-text conversion utilizes specialized medical speech recognition algorithms trained on medical terminology to accurately transcribe spoken medical protocols. The conversion process accounts for variations in pronunciation, accents, and speaking speeds to produce accurate text representations of the spoken protocols. In another embodiment, the speech-to-text conversion may include speaker diarization to distinguish between multiple speakers in a recorded protocol discussion, allowing the system to properly attribute different sections of the protocol to the appropriate healthcare providers.

At operation 216, if the input type is determined to be a plain text document, text extraction is performed. In one embodiment, the text extraction process parses the document to extract the relevant protocol text while removing headers, footers, page numbers, and other non-protocol content. The extraction process preserves the structural elements of the protocol, such as numbered steps, bullet points, and paragraph breaks. In another embodiment, the text extraction may include format conversion from proprietary document formats to a standardized text format that can be more easily processed by subsequent natural language processing operations.

After performing one of the conversion operations (212, 214, or 216), the method 200 proceeds to operation 218 where the converted text is preprocessed and sanitized. In one embodiment, preprocessing includes removing extraneous characters, correcting common OCR or speech-to-text errors, and normalizing text formatting. Sanitization involves removing personally identifiable information that might have been included in the protocol, ensuring compliance with privacy regulations. In another embodiment, preprocessing may include sentence boundary detection and paragraph segmentation to preserve the logical structure of the protocol, while sanitization may involve identifying and flagging potential inconsistencies or ambiguities in the protocol text for later review.

The preprocessing and sanitization at operation 218 improves the accuracy of subsequent analysis by ensuring consistent text formatting and removing noise that could interfere with natural language processing. This technical improvement enhances the reliability of the protocol digitization process and reduces the computational resources required for later processing stages.

At operation 220, the method 200 includes standardizing medical terminology in the preprocessed text. In one embodiment, standardization involves mapping variant terms, abbreviations, and acronyms to their canonical representations using a medical terminology dictionary. For example, "BP" might be standardized to "blood pressure" and "MI" to "myocardial infarction." In another embodiment, the standardization process may use context-aware algorithms to disambiguate terms that have multiple meanings depending on their usage context, ensuring that the correct standardized term is selected based on the surrounding text.

The standardization of medical terminology creates a consistent vocabulary throughout the protocol, which facilitates more accurate identification of actions and decisions in subsequent processing steps. This technical advantage enables more efficient data storage and retrieval compared to maintaining multiple variant terms for the same medical concept.

At operation 222, the method 200 includes performing natural language processing (NLP) analysis on the standardized text using trained models. In one embodiment, the NLP analysis utilizes a combination of rule-based approaches and machine learning models trained specifically on annotated medical protocols. These models are designed to understand the unique structure and language patterns of medical protocols, allowing for more accurate extraction of key elements. In another embodiment, the NLP analysis may employ deep learning techniques such as transformer-based language models that have been fine-tuned on medical protocol corpora to capture complex linguistic patterns and domain-specific knowledge.

Following operation 222, the method 200 includes three parallel operations: operation 224 for identifying action nodes, operation 226 for identifying decision nodes, and operation 228 for determining node relationships. These operations may be performed simultaneously to improve processing efficiency.

At operation 224, the method 200 includes identifying action nodes representing medical actions. In one embodiment, action nodes are identified using named entity recognition (NER) techniques that detect verbs and noun phrases associated with medical procedures, medication administration, and patient monitoring tasks. For example, phrases like "administer epinephrine" or "monitor blood pressure" would be identified as action nodes. In another embodiment, the action node identification may include extraction of associated parameters such as dosage information, timing requirements, and specific techniques for performing the actions.

At operation 226, the method 200 includes identifying decision nodes representing conditional branches in the protocol. In one embodiment, decision nodes are identified by detecting conditional statements, such as "if-then" constructs, and threshold values for patient vital signs or laboratory results. For example, "if blood pressure is below 90/60 mmHg, then..." would be identified as a decision node. In another embodiment, the decision node identification may include extraction of complex conditional logic involving multiple parameters and Boolean operators, allowing the system to represent complex clinical decision-making processes.

At operation 228, the method 200 includes determining relationships between the identified action and decision nodes. In one embodiment, relationships are determined based on textual indicators such as sequential numbering, temporal adverbs (e.g., "then," "after"), and causal conjunctions (e.g., "because," "therefore"). The system analyzes sentence structure and paragraph organization to establish the flow of the protocol. In another embodiment, the relationship determination may include identification of parallel paths, loops, and exception handling in the protocol, enabling the representation of complex protocol structures that may not follow a simple linear progression.

The parallel processing of operations 224, 226, and 228 enables more efficient extraction of protocol structure from the text, reducing the overall processing time compared to sequential analysis. This technical improvement enhances the scalability of the protocol digitization system when processing large volumes of medical protocols.

At operation 230, the method 200 includes generating a protocol graph structure based on the identified nodes and relationships. In one embodiment, the protocol graph structure is created as a directed graph where nodes represent action and decision points, and edges represent transitions between nodes. Each node includes a position identifier and a parent position identifier to establish a hierarchical structure. In another embodiment, the protocol graph generation may include optimization of the graph structure to minimize redundancy and increase computational efficiency during traversal, such as merging similar nodes or creating shortcut edges for frequently traversed paths.

The generated protocol graph structure provides a digital representation of the medical protocol that enables automated navigation and integration with patient data. This technical advantage allows for automated traversal algorithms when determining appropriate care paths based on patient data, improving the efficiency of protocol execution.

At operation 232, the method 200 includes validating the protocol graph structure. In one embodiment, validation involves verifying that all decision nodes have corresponding action nodes, checking for disconnected nodes that cannot be reached from the starting point, and identifying circular references that could cause infinite loops during execution. In another embodiment, the validation process may include verification of clinical parameters against medical databases to ensure that threshold values and dosage recommendations fall within accepted ranges, reducing the risk of digitizing protocols with potentially dangerous instructions.

The validation of the protocol graph structure ensures the integrity and safety of the digitized protocol, providing a technical improvement over manual protocol management where such validation would be difficult or impossible to perform systematically.

At operation 234, the method 200 includes storing the validated protocol graph structure in a database. In one embodiment, the protocol graph structure is stored in a structured format that preserves the nodes, edges, position identifiers, and parent-child relationships. The database schema is adjusted for efficient retrieval and traversal of the protocol graph. In another embodiment, the storage process may include indexing of the protocol graph based on medical conditions, procedures, and keywords to facilitate rapid search and retrieval when needed for patient care.

The structured storage of the protocol graph reduces computational overhead associated with document management and enables more efficient data retrieval compared to traditional document formats. This technical advantage supports the system's ability to quickly access relevant protocols based on patient conditions.

At operation 236, the method 200 includes generating a visual representation of the protocol graph structure. In one embodiment, the visual representation displays the protocol as a flowchart with distinct visual styles for action nodes, decision nodes, and the current position within the protocol. The visualization includes interactive elements that allow healthcare providers to navigate through the protocol and input patient data at decision points. In another embodiment, the visual representation may include multiple view options, such as a condensed overview for quick reference and a detailed view showing all parameters and instructions associated with each node.

The visual representation of the protocol graph structure enhances the usability of the digitized protocol by providing clear guidance to healthcare providers and reducing the cognitive load associated with following complex medical protocols. This technical improvement supports better adherence to established care standards and reduces the risk of errors in protocol execution.

The method 200 for digitizing medical protocols provides technical advantages over existing approaches to medical protocol management. By automating the conversion of unstructured protocols into structured digital formats, the method reduces manual effort, improves consistency, and enables integration with hospital information systems. The graph-based representation allows for dynamic navigation based on patient data, supporting more personalized and efficient patient care while maintaining detailed documentation for regulatory compliance and quality assurance purposes.

Referring to FIG. 3, a natural language processing pipeline 304 for converting preprocessed medical protocol text into a protocol graph structure is shown. The natural language processing pipeline 304 receives preprocessed medical protocol text 302 as input and processes this text through multiple specialized modules to generate a protocol graph structure 318 as output. The preprocessed medical protocol text 302 represents medical protocols that have been converted from various unstructured formats, such as handwritten documents, audio recordings, or plain text documents, into a standardized text format with consistent terminology. The natural language processing pipeline 304 applies a series of analytical processes to transform this preprocessed text into a structured representation that can be integrated with hospital information systems and used to guide healthcare providers through appropriate care pathways.

The natural language processing pipeline 304 includes a named entity recognition module 306 configured to identify and classify a predetermined number of element types within the preprocessed medical protocol text 302. In one embodiment, the named entity recognition module 306 employs a machine learning model trained on annotated medical protocols to recognize specific types of medical entities, such as medications, procedures, vital signs, and laboratory tests. The named entity recognition module 306 analyzes the linguistic patterns, contextual cues, and domain-specific terminology within the preprocessed text to identify relevant entities. In another embodiment, the named entity recognition module 306 utilizes a hybrid approach combining rule-based pattern matching with statistical models to improve recognition accuracy for specialized medical terminology. The named entity recognition module 306 is operatively connected to both the action node identification 308 and decision node identification 310 components, providing them with the classified entities for further processing and categorization.

The action node identification 308 component of the named entity recognition module 306 is configured to identify medical actions within the preprocessed protocol text. In one embodiment, the action node identification 308 component detects verbs and associated noun phrases that represent specific medical procedures, medication administration tasks, or patient monitoring activities. For example, phrases such as "administer epinephrine," "monitor blood pressure," or "perform CPR" would be identified as action nodes. In another embodiment, the action node identification 308 component extracts associated parameters for each action, such as dosage information for medications, timing requirements for procedures, or specific techniques for performing the actions. The action node identification 308 component assigns a unique position identifier to each identified action node and determines its hierarchical relationship to other nodes by establishing parent position identifiers. This hierarchical structure enables the system to represent complex medical protocols with multiple levels of nested actions and decisions.

The decision node identification 310 component of the named entity recognition module 306 is configured to identify conditional branches within the preprocessed protocol text. In one embodiment, the decision node identification 310 component detects conditional statements, such as "if-then" constructs, and threshold values for patient vital signs or laboratory results. For example, a statement like "if blood pressure is below 90/60 mmHg, then administer fluid resuscitation" would be identified as a decision node with a specific threshold value and associated action. In another embodiment, the decision node identification 310 component extracts complex conditional logic involving multiple parameters and Boolean operators, allowing the system to represent nuanced clinical decision-making processes. The decision node identification 310 component works in conjunction with the action node identification 308 component to establish the logical flow of the medical protocol, determining which actions should be performed based on specific patient conditions or clinical findings.

The relationship extraction 312 component of the natural language processing pipeline 304 is configured to determine the connections and dependencies between the identified action and decision nodes. In one embodiment, the relationship extraction 312 component analyzes textual indicators such as sequential numbering, temporal adverbs (e.g., "then," "after"), and causal conjunctions (e.g., "because," "therefore") to establish the flow of the protocol. The component examines sentence structure and paragraph organization to determine the order in which actions should be performed and how decision outcomes affect the protocol path. In another embodiment, the relationship extraction 312 component identifies parallel paths, loops, and exception handling in the protocol, enabling the representation of complex protocol structures that may not follow a simple linear progression. The relationship extraction 312 component creates edges between nodes based on the identified temporal and causal relationships, forming the basic structure of the protocol graph. These edges represent transitions between nodes and define the possible paths through the medical protocol based on different patient conditions or clinical scenarios.

The confidence scoring 314 component of the natural language processing pipeline 304 is configured to assess the reliability of the identified nodes and relationships. In one embodiment, the confidence scoring 314 component assigns numerical values to each identified entity, action, decision, and relationship based on the certainty of the identification. These scores are calculated using factors such as the clarity of the language in the original text, the consistency with known medical terminology, and the statistical likelihood based on the training data. In another embodiment, the confidence scoring 314 component implements a threshold-based filtering system that flags low-confidence identifications for human review, ensuring that only reliable information is included in the final protocol graph structure. The confidence scoring 314 component is operatively connected to the validation module 316, providing it with scored nodes and relationships for further verification and refinement. This scoring mechanism enhances the overall accuracy of the protocol digitization process by distinguishing between high-confidence identifications that can be automatically processed and low-confidence identifications that require additional verification.

The validation module 316 of the natural language processing pipeline 304 is configured to verify the integrity and clinical validity of the generated protocol structure. In one embodiment, the validation module 316 performs structural validation by verifying that each decision node has at least one corresponding action nodes, checking for disconnected nodes that cannot be reached from the starting point, and identifying circular references that could cause infinite loops during execution. The validation module 316 ensures that the protocol graph adheres to a logical structure that can be traversed without errors or dead ends. In another embodiment, the validation module 316 performs clinical validation by verifying threshold values and ranges against medical databases to ensure that they fall within accepted clinical parameters. For example, it might check that medication dosages are within safe ranges or that vital sign thresholds are clinically appropriate. The validation module 316 generates validation error reports for any issues detected, allowing for correction before the protocol graph structure is finalized. This validation process ensures that the digitized protocols are both structurally sound and clinically accurate, reducing the risk of errors when the protocols are implemented in patient care.

The protocol graph structure 318 represents the output of the natural language processing pipeline 304, providing a structured digital representation of the medical protocol. The protocol graph structure 318 consists of nodes representing action and decision points, with each node including a position identifier and a parent position identifier to establish a hierarchical structure. The edges between nodes represent transitions based on the temporal and causal relationships identified during processing. In one embodiment, the protocol graph structure 318 includes metadata for each node, such as the original text from which it was derived, confidence scores, and any parameters or thresholds associated with the node. In another embodiment, the protocol graph structure 318 incorporates mappings between clinical parameters in decision nodes and corresponding fields in hospital information systems, facilitating integration with patient data. The protocol graph structure 318 is stored in a database for later retrieval and use, providing a digital representation of the medical protocol that enables automated navigation and integration with patient data.

The natural language processing pipeline 304 enables automation of the extraction of structured information from unstructured medical protocols. The pipeline reduces the time and effort required to digitize protocols while improving consistency and accuracy. The graph-based representation enables computational analysis of protocols that would be impossible with traditional document formats, such as automatic detection of conflicts between protocols. The structured representation also allows for automated traversal algorithms when determining appropriate care paths based on patient data, improving the efficiency of protocol execution.

Referring to FIG. 4, a flowchart of a method 400 for validating and storing a protocol graph structure is shown. The method 400 provides ensures the integrity and clinical validity of protocol graph structures generated from unstructured medical protocols. The method 400 begins at operation 402 where nodes are created with position identifiers and parent position identifiers.

At operation 402, the method 400 includes creating nodes with position identifiers and parent position identifiers. In one embodiment, each node in the protocol graph structure is assigned a unique position identifier that distinguishes it from other nodes in the graph. The position identifier may be implemented as a numerical value, alphanumeric string, or other unique identifier that enables efficient traversal and reference of the protocol graph structure. Additionally, each node is assigned a parent position identifier that establishes its hierarchical relationship to other nodes in the graph. For example, a decision node may have a parent position identifier that references an action node that precedes it in the protocol flow. In another embodiment, the creation of nodes may include categorizing each node as either an action node representing a specific medical procedure or a decision node representing a conditional branch in the protocol. The action nodes may include information such as medication administration instructions, patient monitoring tasks, or specific medical procedures to be performed, while decision nodes may include threshold values for patient vital signs or laboratory results that determine the path through the protocol.

After creating nodes with position identifiers and parent position identifiers, the method 400 proceeds to operation 404 where edges between nodes are established based on temporal and causal relationships. In one embodiment, the edges represent transitions between nodes and are created based on the temporal and causal relationships identified during natural language processing of the unstructured medical protocol. For example, textual indicators such as sequential numbering, temporal adverbs (e.g., "then," "after"), and causal conjunctions (e.g., "because," "therefore") in the original protocol text may be used to determine the appropriate connections between nodes. In another embodiment, the establishment of edges may include defining the direction of each edge to represent the flow of the protocol, with each edge connecting a source node to a destination node. The edges may also include metadata such as transition conditions, probability weights, or timing requirements that provide additional context for the relationship between connected nodes. The establishment of edges creates the basic structure of the protocol graph, defining the possible paths through the medical protocol based on different patient conditions or clinical scenarios.

At operation 406, the method 400 includes verifying that all decision nodes have corresponding action nodes. In one embodiment, this verification process ensures that each decision node in the protocol graph structure has at least one action node that can be executed based on the outcome of the decision. For example, if a decision node evaluates whether a patient's blood pressure is below a certain threshold, there should be corresponding action nodes for both the positive and negative outcomes of this evaluation. In another embodiment, the verification process may include checking that each decision node has clearly defined criteria for evaluation and that these criteria are mapped to specific patient parameters that can be measured or observed. This verification helps ensure that the protocol graph structure provides clear guidance for healthcare providers in all possible scenarios and prevents situations where a decision is made but no corresponding action is specified.

Following the verification of decision nodes, the method 400 proceeds to operation 408 where a check for disconnected nodes is performed. In one embodiment, this check identifies any nodes in the protocol graph structure that cannot be reached from the starting point of the protocol. Disconnected nodes may result from errors in the natural language processing of the original protocol text or from incomplete information in the unstructured medical protocol. For example, a section of the protocol that is referenced but not fully described in the original text might result in disconnected nodes in the graph structure. In another embodiment, the check for disconnected nodes may include verifying that each node has at least one incoming edge (except for the starting node) and at least one outgoing edge (except for terminal nodes). This ensures that the protocol graph structure forms a connected graph where all nodes are reachable through some path from the starting point, providing a complete representation of the medical protocol.

At operation 410, the method 400 includes identifying circular references in the protocol graph structure. In one embodiment, circular references are detected by traversing the graph and checking for paths that return to a previously visited node, creating a loop in the protocol flow. Circular references can cause infinite loops during protocol execution, potentially leading to errors in patient care or system failures. For example, if node A leads to node B, which leads to node C, which then leads back to node A, this would constitute a circular reference. In another embodiment, the identification of circular references may include distinguishing between intentional loops in the protocol (such as repeated monitoring of a patient parameter until a certain condition is met) and unintentional circular references that result from errors in the protocol structure. This distinction may be made based on specific markers in the original protocol text or through analysis of the node types and relationships involved in the potential circular reference.

After identifying circular references, the method 400 proceeds to operation 412 where a determination is made regarding whether the validation was successful. In one embodiment, the validation is considered successful if no issues were identified during the verification of decision nodes, the check for disconnected nodes, and the identification of circular references. If the validation is not successful, the method 400 proceeds to operation 414 where a validation error report is generated. In another embodiment, the determination of validation success may include additional criteria such as verifying that the protocol graph structure includes a clear starting point and at least one terminal node, checking that all required metadata for nodes and edges is present, and ensuring that the graph structure accurately represents the original medical protocol. This comprehensive validation helps ensure that the digitized protocol will function correctly when integrated with hospital information systems and used in patient care.

At operation 414, if the validation is not successful, the method 400 includes generating a validation error report. In one embodiment, the validation error report includes detailed information about each issue identified during the validation process, including the type of issue, the specific nodes or edges involved, and recommendations for resolving the issue. For example, if a disconnected node is identified, the report might include the position identifier of the disconnected node and suggest potential parent nodes to which it could be connected. In another embodiment, the validation error report may be categorized by severity level, distinguishing between critical errors that must be resolved before the protocol graph structure can be used and warnings that indicate potential issues that should be reviewed but do not necessarily prevent the use of the protocol. The validation error report provides valuable feedback for improving the quality and reliability of the protocol graph structure before it is integrated with hospital information systems.

If the validation is successful at operation 412, the method 400 proceeds to operation 416 where clinical parameters in the protocol graph structure are mapped to hospital information system fields. In one embodiment, this mapping process establishes connections between the parameters referenced in decision nodes (such as vital signs, laboratory values, or medication information) and the corresponding fields in the hospital information system where this data is stored. For example, a decision node that evaluates a patient's blood pressure would be mapped to the blood pressure fields in the hospital information system. In another embodiment, the mapping process may include creating a data dictionary that defines canonical representations for clinical parameters, standardized units for measurements, and hierarchical relationships between clinical concepts. This mapping enables the automated flow of patient data from the hospital information system to the protocol graph structure, allowing for real-time updates to the protocol position based on changes in patient state.

At operation 418, the method 400 includes validating threshold values and ranges against a medical database. In one embodiment, this validation process compares the threshold values specified in decision nodes (such as normal ranges for vital signs or laboratory values) against established medical guidelines and standards to ensure they fall within clinically appropriate ranges. For example, if a decision node specifies a threshold for high blood pressure, this threshold would be compared against recognized medical standards for hypertension. In another embodiment, the validation of threshold values may include checking for consistency across related protocols and verifying that the units of measurement are clearly specified and standardized. This validation helps ensure that the digitized protocols provide safe and appropriate guidance for patient care, reducing the risk of errors due to incorrect threshold values.

Finally, at operation 420, the method 400 includes storing the validated protocol graph structure in a database. In one embodiment, the protocol graph structure is stored in a structured format that preserves the nodes, edges, position identifiers, and parent-child relationships, as well as any metadata associated with the protocol. The database schema is optimized for efficient retrieval and traversal of the protocol graph during patient care. In another embodiment, the storage process may include indexing the protocol graph based on medical conditions, procedures, and keywords to facilitate rapid search and retrieval when needed for patient care. The stored protocol graph structure serves as a template that can be instantiated for specific patients, allowing for personalized protocol execution while maintaining consistency in the underlying medical guidance.

The method 400 for validating and storing protocol graph structures provides technical advantages over existing approaches to medical protocol management. By verifying the integrity of the protocol graph structure, the method reduces the risk of errors in protocol execution and improves the reliability of the digitized protocols. The mapping of clinical parameters to hospital information system fields enables automated data exchange, reducing manual data entry and the associated error rates. The validation of threshold values against medical databases ensures that the digitized protocols provide clinically appropriate guidance, enhancing patient safety. These technical improvements support the system's ability to provide dynamic guidance to healthcare providers based on patient-specific data while maintaining adherence to established medical standards.

Referring to FIG. 5, a flowchart of a method 500 for establishing and operating a hospital information system connection is shown. The method 500 provides an approach for integrating a medical protocol digitization system with existing hospital information systems to enable real-time patient data exchange and automated protocol navigation. The method 500 begins at operation 502 where a connection with a hospital information system is initialized.

At operation 502, the method 500 includes initializing a hospital information system (HIS) connection. In one embodiment, initializing the HIS connection involves establishing a secure network connection between the medical protocol digitization system 100 (shown in FIG. 1) and the hospital information system 116. The initialization process may include identifying available hospital information systems on the network, selecting the appropriate system based on predefined configuration settings, and establishing communication protocols for data exchange. In another embodiment, the initialization process may include loading connection parameters from a configuration file that specifies network addresses, port numbers, and communication protocols specific to the hospital's infrastructure. The HIS integration module 108 may perform handshaking procedures with the hospital information system 116 to verify that both systems are ready to exchange data and to negotiate communication parameters such as data transfer rates and packet sizes.

After initializing the HIS connection, the method 500 proceeds to operation 504 where system access is authenticated. In one embodiment, authenticating system access involves verifying the credentials of the medical protocol digitization system 100 with the hospital information system 116 using secure authentication protocols such as OAuth, SAML, or Kerberos. The authentication process may include exchanging digital certificates, verifying digital signatures, or using username and password combinations stored in encrypted format. In another embodiment, the authentication process may implement multi-factor authentication that requires both digital credentials and physical confirmation from authorized hospital personnel before granting access to patient data. The authentication process ensures that only authorized systems can access sensitive patient information, maintaining compliance with healthcare privacy regulations such as HIPAA.

At operation 506, the method 500 includes configuring data mappings between the protocol graph structure and the hospital information system fields. In one embodiment, configuring data mappings involves creating a correspondence table that links clinical parameters referenced in decision nodes of the protocol graph structure to specific fields in the hospital information system database where this data is stored. For example, a decision node that evaluates a patient's blood pressure would be mapped to the blood pressure fields in the hospital information system. In another embodiment, the configuration process may include creating a data dictionary that defines canonical representations for clinical parameters, standardized units for measurements, pre-determined ranges for vital signs, and hierarchical relationships between clinical concepts. This mapping enables the automated flow of patient data from the hospital information system to the protocol graph structure, allowing for real-time updates to the protocol position based on changes in patient state.

Following the configuration of data mappings, the method 500 proceeds to operation 508 where real-time data channels are established. In one embodiment, establishing real-time data channels involves creating persistent connections between the medical protocol digitization system 100 and the hospital information system 116 for continuous data streaming. These channels may be implemented using technologies such as WebSockets, MQTT, or HL7 FHIR subscriptions that support real-time notifications when patient data changes. In another embodiment, the establishment of real-time data channels may include configuring event listeners that subscribe to specific patient data events in the hospital information system and trigger callbacks in the medical protocol digitization system when relevant data is updated. The real-time data channels enable the medical protocol digitization system to receive immediate updates about changes in patient state, ensuring that protocol navigation is based on the most current information available.

At operation 510, the method 500 includes beginning continuous operation of the integration between the medical protocol digitization system and the hospital information system. In one embodiment, beginning continuous operation involves activating monitoring processes that maintain the connection between systems, handle communication errors, and automatically reconnect if the connection is interrupted. The system may enter a steady state where it continuously polls for new patient data or waits for event notifications from the established data channels. In another embodiment, beginning continuous operation may include initializing background processes that periodically verify the integrity of the connection, check for system updates, and optimize data transfer rates based on network conditions. This continuous operation ensures that the integration between the medical protocol digitization system and the hospital information system remains robust and responsive throughout patient care.

After beginning continuous operation, the method 500 proceeds to operation 512 where patient data is received from the hospital information system. In one embodiment, receiving patient data involves accepting data packets from the hospital information system that contain updated information about patient vital signs, laboratory results, medication administration records, or other clinical parameters relevant to the medical protocol. The data may be received in response to specific queries from the medical protocol digitization system or as part of a continuous data stream from the established real-time channels. In another embodiment, receiving patient data may include filtering the incoming data stream to identify only the parameters that are relevant to the active protocol graph structures, reducing the processing load and focusing on clinically significant information. The received patient data provides the basis for automated navigation through the protocol graph structure based on the patient's current clinical state.

At operation 514, the method 500 includes validating the format of the received patient data. In one embodiment, validating data format involves checking that the received data conforms to expected structures, contains all required fields, and includes appropriate metadata such as timestamps and measurement units. The validation process may include verifying that numerical values fall within physically possible ranges, checking that categorical data matches predefined value sets, and ensuring that timestamps are logically consistent with the patient's care timeline. In another embodiment, the validation process may implement more sophisticated checks that consider the clinical context of the data, such as verifying that combinations of vital signs are physiologically plausible or that medication dosages are appropriate for the patient's weight and condition. This validation ensures that only reliable data is used for protocol navigation, reducing the risk of errors in patient care.

Following the validation of data format, the method 500 proceeds to operation 516 where the validated data is converted to a canonical format. In one embodiment, converting to canonical format involves transforming the received data into standardized representations that can be consistently processed by the medical protocol digitization system, regardless of the original format from the hospital information system. This may include normalizing units of measurement (e.g., converting all temperature readings to Celsius), standardizing terminology using controlled vocabularies such as SNOMED CT or LOINC, and structuring the data according to a consistent schema. In another embodiment, the conversion process may include enriching the data with additional context derived from medical knowledge bases, such as adding normal range information for laboratory values or calculating derived parameters such as body mass index from height and weight measurements. The canonical format enables consistent evaluation of decision criteria across different protocols and hospital systems.

At operation 518, the method 500 includes updating a state cache with the processed patient data. In one embodiment, updating the state cache involves storing the most recent values of all relevant patient parameters in a high-performance memory structure that enables rapid access during protocol evaluation. The state cache may be organized to optimize lookup operations based on parameter identifiers and may include both current values and historical trends for each parameter. In another embodiment, the state cache may implement a time-series database structure that maintains a chronological record of parameter values, enabling temporal reasoning about the patient's condition and supporting the evaluation of trend-based decision criteria in the protocol graph structure. The state cache provides a computational advantage by reducing the need to repeatedly query the hospital information system for the same data, improving the responsiveness of the protocol navigation system.

After updating the state cache, the method 500 proceeds to operation 520 where protocol decision criteria are checked against the current patient state. In one embodiment, checking protocol decision criteria involves evaluating the conditional expressions associated with decision nodes in the active protocol graph structure using the parameter values stored in the state cache. These evaluations may include simple threshold comparisons (e.g., is blood pressure below 90/60 mmHg?), trend analyses (e.g., has oxygen saturation decreased by more than 5% in the last hour?), or complex logical combinations of multiple parameters. In another embodiment, the evaluation process may employ fuzzy logic or probabilistic reasoning to handle uncertainty in the patient data, assigning confidence levels to different possible paths through the protocol based on the available evidence. This evaluation determines which path through the protocol graph structure is most appropriate given the patient's current clinical state.

At operation 522, the method 500 includes updating the current position within the protocol graph structure based on the evaluation of decision criteria. In one embodiment, updating the protocol position involves moving the current node pointer from the present node to the next appropriate node based on the outcome of the decision criteria evaluation. This update may trigger notifications to healthcare providers about the change in protocol status and may generate recommendations for the next actions to be taken in patient care. In another embodiment, the update process may include maintaining a history of traversed nodes with timestamps and the specific parameter values that triggered each transition, creating a detailed audit trail of the protocol execution. The updated protocol position provides guidance to healthcare providers about the appropriate next steps in patient care based on established medical protocols and the patient's specific condition.

Following the update of the protocol position, the method 500 proceeds to operation 524 where the state of the protocol instance is synchronized across distributed system components. In one embodiment, synchronizing state involves propagating the updated protocol position and associated patient data to all relevant components of the medical protocol digitization system, ensuring that all parts of the system have a consistent view of the patient's status and protocol progress. This synchronization may include updating user interfaces on multiple devices, refreshing cached data in different system modules, and notifying external systems about the protocol state change. In another embodiment, the synchronization process may implement a distributed consensus algorithm to handle concurrent updates from multiple sources, resolving conflicts and ensuring that all system components converge on a consistent state. This synchronization ensures that all healthcare providers interacting with the system have access to the same, current information about the patient's protocol status.

At operation 526, the method 500 includes logging the transaction details for audit and analysis purposes. In one embodiment, logging the transaction involves recording detailed information about the state update, including the previous and new protocol positions, the patient parameters that triggered the transition, the timestamp of the update, and the identities of any healthcare providers involved in the process. The log entries may be stored in a secure, tamper-evident database that maintains the integrity of the audit trail for regulatory compliance and quality assurance purposes. In another embodiment, the logging process may include categorizing the transaction based on clinical significance, flagging critical transitions for review, and generating aggregated statistics about protocol adherence and outcomes. The transaction logs provide valuable data for retrospective analysis of protocol effectiveness, identification of improvement opportunities, and demonstration of compliance with established care standards.

The method 500 for establishing and operating a hospital information system connection provides technical advantages over existing approaches to medical protocol management. By automating the integration between digitized medical protocols and hospital information systems, the method reduces manual data entry and the associated error rates. The real-time data channels enable immediate updates to protocol positions based on changes in patient state, supporting more responsive and personalized patient care. The validation and canonical conversion of patient data ensures consistent evaluation of protocol decision criteria across different hospital systems and data sources. These technical improvements enhance the reliability and performance of the medical protocol digitization system while reducing the computational resources required for protocol management.

Referring to FIG. 6, a flowchart of a method 600 for creating and managing a protocol instance for a specific patient in a healthcare information system is shown. The method 600 provides an approach for instantiating a protocol graph structure for an individual patient and tracking the progression through the protocol based on patient-specific data. The method 600 begins at operation 602 where a protocol instance is created from a protocol graph structure.

At operation 602, the method 600 includes creating a protocol instance from a protocol graph structure. In one embodiment, creating a protocol instance involves generating a working copy of a protocol graph structure that was previously generated through the digitization process described in relation to FIG. 2. The protocol instance maintains the same nodes, edges, position identifiers, and parent-child relationships as the original protocol graph structure, but allows for patient-specific data to be associated with the structure without modifying the base protocol. In another embodiment, creating the protocol instance may include selecting an appropriate protocol graph structure from a database of available protocols based on a patient's diagnosis, presenting condition, or scheduled procedure. For example, if a patient is admitted with symptoms of anaphylaxis, the system may automatically suggest the anaphylaxis emergency protocol graph structure as a candidate for instantiation. The creation of a protocol instance enables personalized protocol execution while maintaining consistency in the underlying medical guidance across multiple patients.

Following the creation of a protocol instance, the method 600 proceeds to operation 604 where the protocol instance is associated with a patient identifier. In one embodiment, associating the protocol instance with a patient identifier involves linking the protocol instance to a unique patient identification number in the hospital information system, such as a medical record number or admission identifier. This association ensures that all data generated during protocol execution is properly attributed to the correct patient and becomes part of their permanent medical record. In another embodiment, the association process may include recording metadata about the protocol instantiation, such as the timestamp of creation, the healthcare provider who initiated the protocol, and the clinical rationale for selecting this particular protocol. This metadata provides valuable context for later review of protocol adherence and outcomes, supporting quality assurance processes and continuous improvement of care protocols.

At operation 606, the method 600 includes initializing the current position within the protocol graph structure. In one embodiment, initializing the current position involves setting the active node pointer to the starting node of the protocol graph structure, typically representing the first action or decision to be made in the patient care process. The system may visually highlight this starting position in the user interface to indicate to healthcare providers where the protocol begins. In another embodiment, the initialization process may include evaluating any pre-conditions for the protocol based on existing patient data, potentially advancing the current position beyond the starting node if certain conditions are already met or documented. For example, if the protocol requires confirmation of patient identity as the first step and this has already been documented in the hospital information system, the current position may be automatically advanced to the next relevant node.

After initializing the current position, the method 600 proceeds to operation 608 where the protocol instance is linked with patient data in the hospital information system. In one embodiment, linking the protocol instance with patient data involves establishing data mappings between the clinical parameters referenced in decision nodes of the protocol graph structure and the corresponding fields in the patient's electronic health record. For example, if a decision node evaluates a patient's blood pressure, the system creates a link to the blood pressure measurements in the patient's record. In another embodiment, the linking process may include configuring real-time data channels to receive updates from medical devices connected to the patient, such as vital sign monitors or ventilators, enabling automatic evaluation of decision criteria based on continuously monitored parameters. This linking of protocol instances with patient data enables the automated flow of information between the protocol execution system and the hospital information system, reducing manual data entry and the associated risk of transcription errors.

At operation 610, the method 600 includes receiving patient state data from the hospital information system. In one embodiment, receiving patient state data involves accepting data packets from the hospital information system that contain updated information about patient vital signs, laboratory results, medication administration records, or other clinical parameters relevant to the medical protocol. The data may be received in response to specific queries from the protocol management system or as part of a continuous data stream from established real-time channels. In another embodiment, receiving patient state data may include filtering the incoming data stream to identify only the parameters that are relevant to the active protocol graph structure, reducing the processing load and focusing on clinically significant information. The received patient state data provides the basis for automated navigation through the protocol graph structure based on the patient's current clinical state.

Following the receipt of patient state data, the method 600 proceeds to operation 612 where a determination is made regarding whether an override is required. In one embodiment, this determination involves evaluating whether the automated protocol navigation based on patient data should be overridden by a healthcare provider due to special circumstances or clinical judgment. For example, a healthcare provider might determine that despite meeting certain criteria for one path in the protocol, the patient's overall condition warrants taking a different path. In another embodiment, the determination may be based on automated detection of unusual or conflicting data patterns that suggest the need for human intervention before proceeding with the protocol. If an override is required, the method 600 proceeds to operation 614; otherwise, it proceeds to operation 616.

At operation 614, if an override is required, the method 600 includes performing a manual override of the protocol navigation. In one embodiment, performing a manual override involves providing an interface for healthcare providers to select an alternative path through the protocol graph structure, bypassing the automated decision logic based on their clinical judgment. The system requires documentation of the reasons for the override and may implement an authorization process based on provider credentials for certain critical deviations from the standard protocol. In another embodiment, the manual override process may include suggesting alternative paths based on secondary parameters or historical outcomes in similar cases, assisting the healthcare provider in making an informed decision about the override. The manual override capability ensures that the protocol system remains a decision support tool rather than replacing clinical judgment, allowing for appropriate flexibility in patient care while maintaining documentation of deviations from standard practice.

If no override is required at operation 612 or after performing a manual override at operation 614, the method 600 proceeds to operation 616 where the current position within the protocol graph structure is updated based on patient data. In one embodiment, updating the current position involves moving the active node pointer from the present node to the next appropriate node based on the evaluation of decision criteria using the received patient data. This update may trigger notifications to healthcare providers about the change in protocol status and may generate recommendations for the next actions to be taken in patient care. In another embodiment, the update process may include calculating confidence scores for different possible paths based on the available patient data, particularly in cases where the data is incomplete or ambiguous, and selecting the path with the highest confidence score. The updated protocol position provides guidance to healthcare providers about the appropriate next steps in patient care based on established medical protocols and the patient's specific condition.

At operation 618, the method 600 includes recording a timestamp and provider notes for the traversed node. In one embodiment, this recording process involves creating a detailed audit trail of each node traversal, including the exact time when the node was activated or completed, the identity of the healthcare provider responsible for the actions at that node, and any clinical observations or notes added by the provider. In another embodiment, the recording process may include capturing parameter values that triggered decision node transitions, creating a comprehensive record of the clinical reasoning behind the protocol progression. This detailed documentation supports regulatory compliance, quality assurance reviews, and potential legal requirements for demonstrating adherence to established care standards.

Following the recording of timestamps and notes, the method 600 proceeds to operation 620 where a determination is made regarding whether a terminal node has been reached. In one embodiment, this determination involves checking whether the current position corresponds to a node that has been designated as a terminal or end point in the protocol graph structure, indicating the completion of the protocol. Terminal nodes may represent successful completion of the protocol, transfer of the patient to another care protocol, or other defined endpoints in the care process. In another embodiment, the determination may include evaluating whether all required documentation for the protocol has been completed, ensuring that the protocol is not considered finished until all necessary information has been recorded. If a terminal node has been reached, the method 600 proceeds to operation 622; otherwise, it returns to operation 610 to continue receiving and processing patient state data.

At operation 622, if a terminal node has been reached, the method 600 includes archiving the protocol instance. In one embodiment, archiving the protocol instance involves storing the complete record of the protocol execution, including all traversed nodes, timestamps, provider notes, and patient data that influenced decision points, in a secure, long-term storage system. The archived protocol instance becomes part of the patient's permanent medical record and may be referenced for future care decisions, quality reviews, or research purposes. In another embodiment, the archiving process may include generating a summary report of the protocol execution, highlighting key decision points, deviations from the standard path, and outcomes, which can be used for quality improvement initiatives or shared with other healthcare providers involved in the patient's ongoing care.

The method 600 for creating and managing a protocol instance provides technical advantages over existing approaches to medical protocol management. By creating patient-specific instances of protocol graph structures, the method enables personalized protocol execution while maintaining consistency in the underlying medical guidance. The integration with hospital information systems allows for automated updates to protocol positions based on patient data, reducing manual data entry and the associated risk of errors. The comprehensive documentation of protocol traversal, including timestamps, provider notes, and override justifications, supports regulatory compliance and quality assurance processes. These technical improvements enhance the reliability and efficiency of protocol-based care while maintaining the flexibility needed to accommodate individual patient needs and clinical judgment.

Referring to FIG. 7, a flowchart of a method 700 for managing related protocols and protocol updates in a medical protocol digitization system is shown. The method 700 provides an approach for handling multiple concurrent protocol instances for a single patient and detecting potential conflicts between related protocols. The method 700 begins at operation 702 where a new protocol instance is created.

At operation 702, the method 700 includes creating a new protocol instance. In one embodiment, creating a new protocol instance involves generating a working copy of a protocol graph structure that was previously generated through the digitization process described in relation to FIG. 2. The protocol instance maintains the same nodes, edges, position identifiers, and parent-child relationships as the original protocol graph structure, but allows for patient-specific data to be associated with the structure without modifying the base protocol. In another embodiment, creating the new protocol instance may include associating the protocol instance with a patient identifier, initializing the current position within the protocol graph structure, and linking the protocol instance with patient data in the hospital information system 116. This operation corresponds to the protocol instance creation process described in relation to FIG. 6, but focuses specifically on the initialization of a new protocol instance that may potentially interact with other protocols.

After creating a new protocol instance, the method 700 proceeds to operation 704 where related protocols are checked. In one embodiment, checking for related protocols involves searching the database 112 for other protocol instances associated with the same patient identifier and identifying protocols that may interact with the newly created protocol instance. The system may analyze clinical parameters referenced in the protocol graph structures to determine potential relationships between protocols. For example, if the new protocol instance is for managing anaphylaxis and an existing protocol instance for the same patient addresses cardiac monitoring, these would be identified as related because both involve monitoring vital signs and potentially administering medications that could interact. In another embodiment, checking for related protocols may include analyzing a predefined relationship database that maps known interactions between different types of medical protocols based on medical knowledge and best practices.

At operation 706, the method 700 includes registering protocol relationships identified during the check for related protocols. In one embodiment, registering protocol relationships involves creating entries in a relationship table that defines connections between specific nodes of different protocol graph structures. For example, the system might register that a medication administration node in one protocol could affect a vital sign threshold in another protocol. In another embodiment, the registration process may include establishing a hierarchy of protocols, identifying which protocol takes precedence in case of conflicts, and defining synchronization points where updates in one protocol should trigger evaluations in related protocols. This registration of relationships enables the system to monitor for potential conflicts and ensure coordinated care across multiple concurrent treatment protocols.

Following the registration of protocol relationships, the method 700 proceeds to operation 708 where instance monitoring begins. In one embodiment, beginning instance monitoring involves activating continuous observation processes that track changes in all related protocol instances, patient state data, and protocol positions. The monitoring system may establish event listeners that subscribe to updates in any of the related protocols and trigger evaluations when changes occur. In another embodiment, beginning instance monitoring may include initializing a state cache that maintains the current positions within all related protocol graph structures and the patient parameters relevant to decision nodes in those protocols. This monitoring ensures that the system can detect potential conflicts as soon as they arise, rather than after conflicting actions have been taken.

At operation 710, the method 700 includes receiving a protocol update. In one embodiment, receiving a protocol update involves detecting a change in one of the monitored protocol instances, such as a transition to a new node in the protocol graph structure based on patient data or healthcare provider input. The update may include information about the specific node being activated, any actions being performed, and the patient parameters that triggered the transition. In another embodiment, receiving a protocol update may include detecting modifications to the protocol graph structure itself, such as when a healthcare provider adds a new node, modifies decision criteria, or changes the relationships between nodes during protocol execution. These updates represent changes that could potentially affect or conflict with other related protocols.

After receiving a protocol update, the method 700 proceeds to operation 712 where related protocols are checked. In one embodiment, checking related protocols involves examining all protocols that were registered as related to the updated protocol during operation 706. The system retrieves the current state of each related protocol, including active nodes, pending decisions, and relevant patient parameters. In another embodiment, checking related protocols may include evaluating the specific relationships that were registered between the updated node and nodes in other protocols, focusing the analysis on the most directly affected protocols rather than all related protocols. This targeted checking improves the efficiency of conflict detection by prioritizing the most likely sources of conflicts.

At operation 714, the method 700 includes analyzing for conflicts between the protocol update and related protocols. In one embodiment, analyzing for conflicts involves comparing medication dosages, treatment timing requirements, and contraindications between the updated protocol and related protocols. For example, if the update involves administering a medication that is contraindicated with a medication prescribed in a related protocol, this would be identified as a conflict. In another embodiment, the conflict analysis may include evaluating the impact of the update on decision criteria in related protocols, such as whether a medication administered in one protocol might affect vital sign thresholds used for decision-making in another protocol. The analysis may also consider temporal constraints, identifying conflicts when timing requirements between protocol steps are incompatible.

Following the analysis for conflicts, the method 700 proceeds to operation 716 where a determination is made regarding whether conflicts were detected. In one embodiment, this determination involves evaluating the results of the conflict analysis against predefined conflict criteria, such as specific medication interactions, incompatible treatment approaches, or contradictory care instructions. If no conflicts are detected, the method 700 proceeds to operation 718; if conflicts are detected, the method 700 proceeds to operation 720.

At operation 718, if no conflicts are detected, the method 700 includes applying the protocol update. In one embodiment, applying the update involves finalizing the transition to the new node in the protocol graph structure, executing any associated actions, and updating the current position within the protocol. The system may also update the visual representation of the protocol graph structure on the display device 118 to reflect the new position. In another embodiment, applying the update may include propagating relevant information to related protocols, such as updating shared patient parameters or triggering reevaluation of decision nodes that depend on the updated information. This propagation ensures that all related protocols remain synchronized with the latest patient data and protocol states.

At operation 720, if conflicts are detected, the method 700 includes generating an alert about the detected conflicts. In one embodiment, generating an alert involves displaying a notification on the display device 118 that includes identifiers of the conflicting protocol graph structures, specific nodes containing conflicting requirements, and patient parameters related to the identified conflicts. The alert may be color-coded based on the severity of the conflict and may include recommendations for resolving the conflict based on medical best practices. In another embodiment, the alert generation may include notifying specific healthcare providers based on their roles and responsibilities, such as alerting a physician for medication conflicts or a nurse for scheduling conflicts. The alert ensures that healthcare providers are promptly informed of potential issues that could affect patient care.

After generating an alert, the method 700 proceeds to operation 722 where the system waits for resolution of the detected conflicts. In one embodiment, waiting for resolution involves pausing the application of the protocol update until a healthcare provider acknowledges the conflict and provides instructions for how to proceed. The system may display the conflicting protocols side by side on the display device 118, highlighting the specific areas of conflict and providing interface elements for selecting resolution options. In another embodiment, waiting for resolution may include implementing a timeout mechanism that escalates the alert if no resolution is provided within a specified timeframe, ensuring that critical conflicts are not overlooked. This waiting period ensures that conflicts are addressed before potentially contradictory actions are taken.

At operation 724, the method 700 includes applying the resolution provided by healthcare providers. In one embodiment, applying the resolution involves implementing the specific instructions provided for resolving the conflict, such as modifying one or both protocols, establishing a precedence relationship, or creating a new protocol that addresses the specific situation. The system may update the protocol graph structures, node relationships, or decision criteria based on the resolution. In another embodiment, applying the resolution may include documenting the clinical reasoning behind the resolution decision, obtaining authorization based on provider credentials, and updating the relationship registrations to prevent similar conflicts in the future. This application of resolutions ensures that conflicts are addressed in a way that maintains the integrity of patient care while respecting clinical judgment.

Finally, at operation 726, the method 700 includes logging the transaction details for audit and analysis purposes. In one embodiment, logging the transaction involves recording detailed information about the protocol update, any conflicts detected, the resolution applied, and the healthcare providers involved in the process. The log entries may be stored in a secure, tamper-evident database that maintains the integrity of the audit trail for regulatory compliance and quality assurance purposes. In another embodiment, the logging process may include categorizing the transaction based on the type of update and conflict, generating aggregated statistics about conflict patterns, and updating machine learning models used for conflict prediction. The transaction logs provide valuable data for retrospective analysis of protocol interactions, identification of common conflict patterns, and improvement of conflict detection algorithms.

The method 700 for managing related protocols and protocol updates provides technical advantages over existing approaches to medical protocol management. By automatically detecting potential conflicts between related protocols, the method reduces the risk of contradictory treatments and improves patient safety. The registration of protocol relationships enables more efficient monitoring and conflict detection, focusing computational resources on the most likely sources of conflicts. The structured approach to conflict resolution ensures that healthcare providers have the information they need to make informed decisions while maintaining documentation of the resolution process. These technical improvements enhance the reliability and safety of concurrent protocol execution while supporting better coordination among healthcare providers.

Referring to FIG. 8, a flowchart of a method 800 for managing protocol graph structure modifications and version control in a medical protocol digitization system is shown. The method 800 provides an approach for tracking changes to protocol graph structures during execution, maintaining version history, and ensuring proper documentation of modifications. The method 800 begins at operation 802 where a base version of a protocol graph structure is stored.

At operation 802, the method 800 includes storing a base version of a protocol graph structure in a database. In one embodiment, storing the base version involves saving the complete protocol graph structure, including all nodes with their position identifiers and parent position identifiers, edges representing relationships between nodes, and associated metadata such as clinical parameters, threshold values, and decision criteria. The base version serves as a reference point for tracking subsequent modifications and may be designated with a version identifier such as "1.0" or a timestamp indicating when it was established as the base version. In another embodiment, storing the base version may include creating a secure, read-only copy of the protocol graph structure that cannot be directly modified, ensuring that all changes are properly tracked through the version control system. The base version may also include information about the protocol's intended use, applicable patient populations, and references to medical literature or guidelines that informed its development.

After storing the base version, the method 800 proceeds to operation 804 where a modification to the protocol graph structure during protocol execution is detected. In one embodiment, detecting a modification involves identifying when a healthcare provider adds a new node, modifies decision criteria, changes the relationships between nodes, or alters any other aspect of the protocol graph structure during patient care. The system may detect these modifications through user interface actions such as drag-and-drop operations, form submissions, or direct editing of protocol elements. In another embodiment, the detection process may include monitoring both intentional modifications made through the user interface and automatic modifications triggered by system events, such as updates to clinical parameters based on new medical research or integration with updated hospital information system fields.

At operation 806, the method 800 includes recording modification details and the provider ID associated with the modification. In one embodiment, recording modification details involves creating a comprehensive log entry that includes the specific changes made to the protocol graph structure, such as nodes added or removed, changes to decision criteria or thresholds, or modifications to node relationships. The log entry also includes the unique identifier of the healthcare provider who made the modification, their role or credentials, and a timestamp indicating when the modification occurred. In another embodiment, the recording process may include capturing the clinical context in which the modification was made, such as the patient state that prompted the change, any unusual circumstances that necessitated deviation from the standard protocol, or the clinical reasoning behind the modification. This detailed documentation supports regulatory compliance and provides valuable context for later review of protocol modifications.

Following the recording of modification details, the method 800 proceeds to operation 808 where a determination is made regarding whether the modification is approved. In one embodiment, this determination involves evaluating the modification against predefined criteria such as clinical safety guidelines, hospital policies, or regulatory requirements. The approval process may include automated validation checks as well as review by authorized personnel such as department heads or medical directors. In another embodiment, the determination may be based on the credentials of the provider who made the modification, with certain types of changes requiring higher levels of authorization. For example, minor adjustments to timing parameters might be automatically approved when made by attending physicians, while structural changes to the protocol graph might require review by a clinical committee. If the modification is approved, the method 800 proceeds to operation 810; if not approved, the method 800 proceeds to operation 812.

At operation 810, if the modification is approved, the method 800 includes propagating the changes to the base version of the protocol graph structure. In one embodiment, propagating changes involves updating the master protocol graph structure stored in the database with the approved modifications, creating a new version of the protocol that will be used for future patient instances. The propagation process may include merging the changes with any other approved modifications that occurred during the same time period, resolving any conflicts that arise. In another embodiment, the propagation process may include applying the changes selectively to specific categories of protocol instances, such as those for particular patient populations or clinical scenarios, rather than universally updating all instances of the protocol.

At operation 812, if the modification is not approved, the method 800 includes documenting the rejection reason. In one embodiment, documenting the rejection reason involves recording a detailed explanation of why the modification was not approved, such as conflicts with established medical guidelines, potential safety concerns, or inconsistencies with other aspects of the protocol. The documentation includes references to relevant policies or literature supporting the rejection decision. In another embodiment, the documentation process may include providing constructive feedback to the healthcare provider who proposed the modification, suggesting alternative approaches that might address their clinical concerns while meeting approval criteria.

At operation 814, the method 800 includes updating the audit trail with the approval status of the modification. In one embodiment, updating the audit trail involves adding an entry to the modification log that indicates whether the change was approved or rejected, who made the approval decision, and when the decision was made. This creates a complete record of the modification lifecycle from proposal through review to final disposition. In another embodiment, the audit trail update may include linking the modification to related quality assurance processes, such as protocol effectiveness reviews or patient outcome analyses, providing context for how the modification fits into broader quality improvement initiatives.

At operation 816, the method 800 includes maintaining a version history with timestamps for the protocol graph structure. In one embodiment, maintaining version history involves storing each approved version of the protocol graph structure with unique version identifiers and timestamps indicating when each version was created. The version history allows healthcare providers to access previous versions of the protocol for reference or to revert to an earlier version if needed. In another embodiment, the version history maintenance may include storing differential changes rather than complete copies of each version, reducing storage requirements while still maintaining a complete record of how the protocol has evolved over time.

Finally, at operation 818, the method 800 includes notifying relevant users of version changes to the protocol graph structure. In one embodiment, notifying users involves sending automated alerts to healthcare providers who frequently use the protocol, informing them of the specific changes made and how these changes might affect patient care. The notifications may be delivered through the hospital information system, email, or mobile alerts depending on the urgency and significance of the changes. In another embodiment, the notification process may include scheduling training sessions or providing educational materials when significant changes are made to protocols, ensuring that all users understand the new version and can implement it correctly in patient care.

The method 800 for managing protocol graph structure modifications and version control provides technical advantages over existing approaches to medical protocol management. By implementing a structured approach to tracking and approving modifications, the method ensures that changes to protocols are properly documented and validated before implementation. The version control system maintains the integrity of protocol graph structures while allowing for continuous improvement based on clinical experience and new medical knowledge. The comprehensive audit trail supports regulatory compliance and quality assurance processes, providing accountability for all modifications to medical protocols.

Referring to FIG. 9, a flowchart of a method 900 for managing patient state and protocol position updates in a medical protocol digitization system is shown. The method 900 provides an approach for processing patient data, evaluating decision criteria, and updating protocol positions based on changes in patient state. The method 900 begins at operation 902 where a state manager is initialized.

At operation 902, the method 900 includes initializing a state manager. In one embodiment, initializing the state manager involves establishing data structures for storing patient parameters, setting up communication channels with the hospital information system 116 (shown in FIG. 1), and loading configuration settings that define how patient data will be processed and evaluated. The state manager may initialize a local cache for storing the most recent values of patient parameters to enable rapid access during protocol evaluation. In another embodiment, the initialization process may include loading protocol-specific thresholds and ranges from the database 112, establishing event listeners for patient data updates, and configuring logging mechanisms for tracking state changes. The initialization of the state manager prepares the system for receiving and processing patient data, ensuring that all necessary components are ready to evaluate protocol decision criteria and update protocol positions.

After initializing the state manager, the method 900 proceeds to operation 904 where patient data is received. In one embodiment, receiving patient data involves accepting data packets from the hospital information system 116 that contain updated information about patient vital signs, laboratory results, medication administration records, or other clinical parameters relevant to the active medical protocol. The data may be received in response to specific queries from the medical protocol digitization system 100 or as part of a continuous data stream from established real-time channels. In another embodiment, receiving patient data may include filtering the incoming data stream to identify only the parameters that are relevant to the active protocol graph structures, reducing the processing load and focusing on clinically significant information. The received patient data provides the basis for automated navigation through the protocol graph structure based on the patient's current clinical state.

At operation 906, the method 900 includes validating the format of the received patient data. In one embodiment, validating data format involves checking that the received data conforms to expected structures, contains all required fields, and includes appropriate metadata such as timestamps and measurement units. The validation process may include verifying that numerical values fall within physically possible ranges, checking that categorical data matches predefined value sets, and ensuring that timestamps are logically consistent with the patient's care timeline. In another embodiment, the validation process may implement more sophisticated checks that consider the clinical context of the data, such as verifying that combinations of vital signs are physiologically plausible or that medication dosages are appropriate for the patient's weight and condition. This validation ensures that only reliable data is used for protocol navigation, reducing the risk of errors in patient care.

Following the validation of data format, the method 900 proceeds to operation 908 where the state cache is updated with the validated patient data. In one embodiment, updating the state cache involves storing the most recent values of all relevant patient parameters in a high-performance memory structure that enables rapid access during protocol evaluation. The state cache may be organized to optimize lookup operations based on parameter identifiers and may include both current values and historical trends for each parameter. In another embodiment, the state cache may implement a time-series database structure that maintains a chronological record of parameter values, enabling temporal reasoning about the patient's condition and supporting the evaluation of trend-based decision criteria in the protocol graph structure. The state cache provides a computational advantage by reducing the need to repeatedly query the hospital information system for the same data, improving the responsiveness of the protocol navigation system.

At operation 910, the method 900 includes evaluating decision nodes against the current patient state. In one embodiment, evaluating decision nodes involves processing the conditional expressions associated with decision nodes in the active protocol graph structure using the parameter values stored in the state cache. These evaluations may include simple threshold comparisons (e.g., is blood pressure below 90/60 mmHg?), trend analyses (e.g., has oxygen saturation decreased by more than 5% in the last hour?), or complex logical combinations of multiple parameters. In another embodiment, the evaluation process may employ fuzzy logic or probabilistic reasoning to handle uncertainty in the patient data, assigning confidence levels to different possible paths through the protocol based on the available evidence. This evaluation determines which path through the protocol graph structure is most appropriate given the patient's current clinical state.

Following the evaluation of decision nodes, the method 900 proceeds to operation 912 where a determination is made regarding whether a position update is required. In one embodiment, this determination involves checking whether the outcome of the decision node evaluation indicates that the current position within the protocol graph structure should change. If a position update is required, the method 900 proceeds to operation 914; otherwise, it proceeds to operation 920.

At operation 914, if a position update is required, the method 900 includes determining whether an override is active. In one embodiment, determining whether an override is active involves checking whether a healthcare provider has manually overridden the automated protocol navigation system, indicating that the system should not automatically update the protocol position despite changes in patient data. Overrides may be activated when healthcare providers need to maintain a specific protocol position due to clinical considerations not captured in the protocol graph structure. In another embodiment, the determination may include checking for system-level overrides that may be activated during certain procedures or emergency situations where automated updates could interfere with critical care activities. If an override is active, the method 900 proceeds to operation 916; otherwise, it proceeds to operation 918.

At operation 916, if an override is active, the method 900 includes waiting for manual resolution. In one embodiment, waiting for manual resolution involves pausing the automated position update process and displaying a notification to healthcare providers that a potential position update has been identified but not applied due to an active override. The system may provide interface elements for healthcare providers to review the suggested update and either maintain the override or allow the update to proceed. In another embodiment, the waiting process may include implementing a timeout mechanism that escalates the notification if no resolution is provided within a specified timeframe, ensuring that important updates are not indefinitely delayed. After manual resolution is provided, the method 900 proceeds to operation 920.

At operation 918, if no override is active, the method 900 includes updating the position within the protocol graph structure. In one embodiment, updating the position involves moving the current node pointer from the present node to the next appropriate node based on the outcome of the decision criteria evaluation. This update may trigger notifications to healthcare providers about the change in protocol status and may generate recommendations for the next actions to be taken in patient care. In another embodiment, the update process may include maintaining a history of traversed nodes with timestamps and the specific parameter values that triggered each transition, creating a detailed audit trail of the protocol execution. After updating the position, the method 900 proceeds to operation 920.

Finally, at operation 920, the method 900 includes logging the state changes. In one embodiment, logging state changes involves recording detailed information about updates to patient parameters, evaluations of decision criteria, and changes in protocol position. The log entries may include timestamps, parameter values, decision outcomes, and identifiers for healthcare providers involved in any manual interventions. In another embodiment, the logging process may categorize state changes based on clinical significance, flagging critical transitions for review, and generating aggregated statistics about protocol adherence and outcomes. The comprehensive logging of state changes supports regulatory compliance, quality assurance processes, and retrospective analysis of protocol effectiveness.

The method 900 for managing patient state and protocol position updates provides technical advantages over existing approaches to medical protocol management. By maintaining a state cache of patient measurements, the method reduces the computational overhead of repeatedly querying the hospital information system for the same data. The structured evaluation of decision criteria against current patient state enables automated navigation through complex protocol graphs while maintaining the flexibility for healthcare provider overrides when needed. The comprehensive logging of state transitions creates a detailed audit trail that supports regulatory compliance and quality improvement initiatives.

Referring to FIG. 10, a flowchart of a method 1000 for processing protocol visualization and user inputs in a medical protocol digitization system is shown. The method 1000 provides an approach for dynamically updating a user interface based on protocol state changes and processing user interactions with the protocol graph structure. The method 1000 begins at operation 1002 where user interface components are initialized.

At operation 1002, the method 1000 includes initializing user interface (UI) components. In one embodiment, initializing UI components involves loading and configuring the visual elements required to display the protocol graph structure, such as node representations, edge connectors, position indicators, and interactive controls. The initialization process may include setting up event listeners for user interactions, configuring display parameters based on user preferences or device capabilities, and establishing communication channels with other system modules such as the HIS integration module 108 and protocol graph generator 106. In another embodiment, the initialization process may include loading protocol-specific visualization templates that define how different types of nodes and relationships should be visually represented. For example, action nodes might be displayed as rectangular boxes while decision nodes might be displayed as diamond shapes, with different color schemes to indicate node status or importance. The initialization of UI components prepares the system for displaying protocol information and receiving user input, establishing the foundation for interactive protocol navigation.

Following the initialization of UI components, the method 1000 proceeds to operation 1004 where a protocol state update is received. In one embodiment, receiving a protocol state update involves accepting data from the HIS integration module 108 or other system components that indicate changes in the current position within the protocol graph structure, patient parameters relevant to decision nodes, or modifications to the protocol graph structure itself. The update may include information about newly traversed nodes, completed actions, decision outcomes based on patient data, or healthcare provider interventions. In another embodiment, receiving a protocol state update may include detecting changes in related protocols that might affect the display of the current protocol, such as conflicts identified by the method 700 described in relation to FIG. 7. The protocol state update provides the basis for refreshing the visual representation of the protocol graph structure to reflect the current state of patient care.

At operation 1006, the method 1000 includes updating the protocol visualization based on the received state update. In one embodiment, updating the protocol visualization involves redrawing the protocol graph structure to reflect any changes in node status, relationships, or protocol position. This may include adjusting the layout of the graph to optimize visibility of the current position and available paths, applying visual styles to nodes based on their status (e.g., completed, active, pending), and updating any textual information associated with nodes such as parameter values or timestamps. In another embodiment, the update process may include animating transitions between different states of the protocol graph to provide visual cues about the direction and nature of the change, helping healthcare providers maintain context as the protocol progresses. The updated visualization provides healthcare providers with a clear representation of the current state of the protocol and the patient's progression through the care pathway.

After updating the protocol visualization, the method 1000 proceeds to operation 1008 where the current position within the protocol graph structure is highlighted. In one embodiment, highlighting the current position involves applying a distinct visual style to the node representing the current step in the protocol, such as a bright color, bold outline, or pulsating effect that draws attention to this node. The highlighting may also include adjusting the view to center the current position on the display device 118, ensuring that it remains visible even in complex protocol graphs. In another embodiment, the highlighting process may include providing additional contextual information about the current position, such as displaying detailed instructions associated with the current node, showing relevant patient parameters, or indicating how long the current node has been active. This highlighting helps healthcare providers quickly identify the current step in the protocol, reducing the cognitive load associated with following complex medical protocols.

At operation 1010, the method 1000 includes calculating available paths forward from the current position. In one embodiment, calculating available paths involves analyzing the protocol graph structure to identify all possible next nodes that could be traversed based on the current position and patient state. For decision nodes, this may include evaluating each potential branch against current patient parameters to determine which paths are valid or recommended. The calculation may assign probability or confidence scores to different paths based on the available patient data, highlighting the most likely or appropriate next steps. In another embodiment, the calculation process may include identifying shortcuts or alternative paths that could be taken based on healthcare provider input or special circumstances, providing flexibility in protocol execution while maintaining awareness of the standard path. The calculation of available paths enables the system to provide guidance to healthcare providers about potential next steps in patient care based on the protocol graph structure and current patient state.

Following the calculation of available paths, the method 1000 proceeds to operation 1012 where the historical view of previously traversed nodes is updated. In one embodiment, updating the historical view involves maintaining a visual record of the path that has been taken through the protocol graph structure, such as by graying out or applying a distinct color to nodes that have already been traversed. The historical view may include timestamps indicating when each node was active and summary information about actions performed or decisions made at each step. In another embodiment, the update process may include providing interactive elements that allow healthcare providers to review detailed information about previous steps, such as specific parameter values that triggered decisions or notes added by healthcare providers during execution. This historical view supports continuity of care by providing context about the patient's journey through the protocol and facilitating handoffs between different healthcare providers.

At operation 1014, the method 1000 includes determining whether user input has been received. In one embodiment, this determination involves checking for interactions with the user interface, such as clicks on specific nodes, selections from dropdown menus, text entries in form fields, or activation of control buttons. The system may prioritize different types of inputs based on their clinical significance or urgency, ensuring that critical interventions are processed promptly. In another embodiment, the determination may include monitoring for inputs from multiple sources, such as touchscreen interactions, keyboard commands, voice inputs, or signals from connected medical devices that might indicate the need for user attention. If user input is received, the method 1000 proceeds to operation 1016; if no user input is received, the method 1000 proceeds to operation 1024.

At operation 1016, if user input is received, the method 1000 includes determining whether the input represents an override request. In one embodiment, determining whether an override request has been made involves analyzing the nature of the user input to identify actions that would deviate from the standard protocol flow, such as selecting a node that is not on the calculated available paths or explicitly activating an override control. The system may apply different thresholds for identifying overrides based on the clinical context and the potential impact of the deviation. In another embodiment, the determination may include evaluating the user's credentials against authorization requirements for different types of overrides, with more significant deviations requiring higher levels of authorization. If the input represents an override request, the method 1000 proceeds to operation 1020; otherwise, it proceeds to operation 1018.

At operation 1018, if the input is not an override request, the method 1000 includes processing the standard input. In one embodiment, processing standard input involves interpreting user actions within the context of normal protocol execution, such as confirming completion of an action node, providing parameter values for decision nodes, or navigating to the next recommended step in the protocol. The processing may include validation of entered data against expected ranges or formats, providing immediate feedback about any issues detected. In another embodiment, the processing may include contextual assistance based on the current protocol position, such as suggesting appropriate values for parameters or providing reference information relevant to the current step. The processing of standard input enables healthcare providers to interact with the protocol graph structure in a guided manner, supporting adherence to established care standards while maintaining efficiency in documentation.

At operation 1020, if the input represents an override request, the method 1000 includes processing the override. In one embodiment, processing an override involves documenting the nature of the deviation from the standard protocol, capturing the healthcare provider's rationale for the override, and obtaining any required authorizations based on provider credentials and the type of override. The system may implement different levels of documentation requirements based on the clinical significance of the override, with more substantial deviations requiring more detailed justification. In another embodiment, the override processing may include suggesting alternative approaches that might address the healthcare provider's concerns while minimizing deviation from the protocol, or providing warnings about potential risks associated with the override based on medical knowledge bases. The processing of overrides ensures that deviations from standard protocols are properly documented and authorized, supporting clinical flexibility while maintaining accountability.

After processing either standard input or an override, the method 1000 proceeds to operation 1022 where documentation is updated. In one embodiment, updating documentation involves recording detailed information about the user input and system response, including timestamps, user identifiers, specific actions taken, and any data entered or modified. The documentation may be structured to support regulatory compliance requirements and quality assurance processes, creating a comprehensive audit trail of protocol execution. In another embodiment, the documentation update may include generating structured notes that can be incorporated into the patient's electronic health record, summarizing key decisions and interventions in a format that supports clinical communication and continuity of care. The updated documentation provides a record of protocol adherence and any deviations, supporting accountability and enabling retrospective analysis of care quality.

Finally, at operation 1024, the method 1000 includes refreshing the display to reflect the current state of the protocol and any changes resulting from user input. In one embodiment, refreshing the display involves redrawing all visual elements based on the most current data, ensuring that healthcare providers have access to the most up-to-date information about the protocol status and patient state. The refresh process may include optimizing the layout based on the current context, such as expanding details for the active node while collapsing less relevant sections of the protocol graph. In another embodiment, the refresh process may include applying visual transitions or animations that help maintain context when significant changes occur, reducing the cognitive load associated with tracking changes in complex protocols. The refreshed display provides healthcare providers with a clear and current view of the protocol status, supporting informed decision-making and efficient patient care.

The method 1000 for processing protocol visualization and user inputs provides technical advantages over existing approaches to medical protocol management. By dynamically updating the visual representation based on protocol state changes and user interactions, the method reduces the cognitive load on healthcare providers and improves the usability of the medical protocol digitization system. The calculation of available paths based on current patient data enables more informed decision-making, while the comprehensive documentation of user inputs and overrides supports regulatory compliance and quality assurance processes. These technical improvements enhance the system's ability to provide dynamic guidance to healthcare providers while maintaining a detailed record of protocol adherence.

The disclosure also provides support for a method for digitizing medical protocols, comprising: receiving an unstructured medical protocol, converting the unstructured medical protocol into machine-readable text, analyzing the machine-readable text using natural language processing to: identify action nodes representing medical actions, identify decision nodes representing conditional branches, and determine relationships between the action nodes and decision nodes, generating a protocol graph structure based on the identified nodes and relationships, wherein: the protocol graph structure comprises nodes and edges, each node includes a position identifier and a parent position identifier, and the edges represent transitions between nodes, storing the protocol graph structure in a database, and generating a visual representation of the protocol graph structure showing available paths through the medical protocol. In a first example of the method, analyzing the machine-readable text comprises: training a named entity recognition model using annotated medical protocols to identify: medical actions comprising medication administration and patient monitoring tasks, decision criteria comprising threshold values for patient vital signs, and temporal relationships between actions and decisions, and applying the trained named entity recognition model to the machine-readable text. In a second example of the method, optionally including the first example, generating the protocol graph structure comprises: creating nodes comprising position identifiers and parent position identifiers, establishing edges between nodes based on identified temporal and causal relationships, validating the protocol graph structure by: verifying all decision nodes have corresponding action nodes, checking for disconnected nodes, and identifying circular references. In a third example of the method, optionally including one or both of the first and second examples, the method further comprises: receiving patient state data from a hospital information system, mapping the patient state data to decision nodes in the protocol graph structure using a medical terminology dictionary, automatically updating a current position within the protocol graph structure based on the mapped patient state data, and generating alerts when the patient state data triggers a transition between nodes. In a fourth example of the method, optionally including one or more or each of the first through third examples, mapping the patient state data comprises: maintaining a data mapping comprising: canonical representations for clinical parameters, standardized units for measurements, pre-determined ranges for vital signs, and hierarchical relationships between clinical concepts, and converting received patient state data into the canonical representations. In a fifth example of the method, optionally including one or more or each of the first through fourth examples, the method further comprises: creating an instance of the protocol graph structure for a patient, recording timestamps and healthcare provider notes for each traversed node, maintaining an audit trail of override actions and manual position updates, and archiving the instance upon reaching a terminal node. In a sixth example of the method, optionally including one or more or each of the first through fifth examples, generating the visual representation comprises: displaying a current node in a first visual style, displaying available next nodes based on current patient state data, providing interactive elements for healthcare provider input at decision nodes, and maintaining a visual history of traversed nodes.

The disclosure also provides support for a system for digitizing medical protocols, comprising: a computing device comprising: one or more processors, a display device, and a memory storing instructions executable by the one or more processors to: receive an unstructured medical protocol via an input interface, wherein the unstructured medical protocol comprises at least one of handwritten text, plain text, or spoken input, convert the unstructured medical protocol into machine-readable text, analyze the machine-readable text using natural language processing to: identify action nodes representing medical actions to be performed, identify decision nodes representing patient state-dependent branches, and determine relationships between the action nodes and decision nodes, generate a protocol graph structure comprising: nodes representing the identified action nodes and decision nodes, wherein each node includes a position identifier and a parent position identifier, and edges representing the determined relationships between nodes, receive patient state data from a hospital information system, automatically update a current position within the protocol graph structure based on the received patient state data, and output, to the display device, a visual representation of the protocol graph structure showing: available paths through the medical protocol, the current position within the protocol graph structure, and historical progression through previously traversed nodes. In a first example of the system, the instructions are further executable to: create a protocol instance for a patient by: associating the protocol graph structure with a patient identifier, initializing the current position within the protocol graph structure, linking the protocol instance with patient data in the hospital information system, and storing the protocol instance in a database. In a second example of the system, optionally including the first example, the instructions are further executable to: receive real-time patient data from medical devices connected to the hospital information system, automatically map the real-time patient data to decision criteria in the protocol graph structure, update the current position within the protocol graph structure based on the mapped patient data, and generate alerts when the updated position is associated with medical attention. In a third example of the system, optionally including one or both of the first and second examples, updating the current position comprises instructions executable to: maintain a state cache of patient measurements, evaluate decision node criteria against the state cache, synchronize protocol instance states across distributed system components, and log state transitions with associated timestamps. In a fourth example of the system, optionally including one or more or each of the first through third examples, the instructions are further executable to: manage multiple concurrent protocol instances for a single patient by: storing, in a protocol instance database, multiple active protocol graph structures associated with a patient identifier, maintaining a mapping table defining clinical relationships between nodes of different protocol graph structures, automatically updating patient positions within related protocol graph structures when: a measured patient parameter satisfies decision criteria in a first protocol graph structure, and the measured patient parameter is mapped to decision criteria in at least one other protocol graph structure, comparing medication dosages and treatment timing requirements between concurrent protocol graph structures, identifying conflicts when: two or more protocol graph structures specify contradictory medications, or timing requirements between protocol steps are incompatible, and generating, on the display device, an alert comprising: identifiers of the two or more protocol graph structures, specific nodes containing conflicting requirements, and patient parameters related to the identified conflicts. In a fifth example of the system, optionally including one or more or each of the first through fourth examples, the instructions are further executable to: provide an override interface enabling healthcare providers to: manually override automated position updates, document reasons for overrides, specify alternative paths through the protocol graph structure, and obtain authorization for overrides based on provider credentials.

The disclosure also provides support for a method for digitizing and managing medical protocols in a hospital information system (HIS), comprising: preprocessing an unstructured medical protocol by: receiving the unstructured medical protocol in at least one of handwritten, spoken, or plain text format, converting the unstructured medical protocol into machine-readable text, sanitizing the machine-readable text by removing extraneous characters and standardizing medical terminology, analyzing the preprocessed text using a trained natural language processing (NLP) model to: identify action nodes representing specific medical procedures using named entity recognition, identify decision nodes representing patient state-dependent branches by detecting conditional statements and threshold values, determine temporal and causal relationships between the identified nodes based on textual indicators and sentence structure, extract measurement thresholds and clinical parameters associated with the decision nodes, generating a protocol graph structure by: creating nodes comprising position identifiers and parent position identifiers, establishing directed edges between nodes based on the determined relationships, validating the protocol graph structure, mapping clinical parameters in decision nodes to corresponding fields in the hospital information system, creating a protocol instance for a specific patient by: associating the protocol graph structure with a patient identifier, initializing a current position within the protocol graph structure, receiving real-time patient state data from the hospital information system, automatically updating the current position within the protocol graph structure based on the received patient state data and the mapped clinical parameters, storing timestamps, and displaying, on a graphical user interface: a visual representation of the protocol graph structure, the current position within the protocol graph structure, available paths forward based on current patient state data, and historical progression through previously traversed nodes. In a first example of the method, preprocessing the unstructured medical protocol comprises: receiving at least one of: handwritten protocol documents processed using optical character recognition, audio recordings processed using speech-to-text conversion, or plain text documents, standardizing medical terminology using a medical terminology dictionary, and filtering characters and formatting. In a second example of the method, optionally including the first example, analyzing the preprocessed text comprises: training a named entity recognition model using annotated medical protocols to identify: medical measurements and their associated units, medication names and dosages, temporal relationships between protocol steps, and conditional statements linking patient states to medical actions, wherein the trained model assigns confidence scores to identified entities. In a third example of the method, optionally including one or both of the first and second examples, validating the protocol graph structure comprises: verifying that each decision node includes: at least one input parameter mapped to a hospital information system field, threshold values or ranges for the input parameter, and at least two output paths corresponding to different parameter values, checking for disconnected nodes and circular references, and validating medication dosages against a medical database. In a fourth example of the method, optionally including one or more or each of the first through third examples, the method further comprises: maintaining version control of protocol graph structures by: storing a base version of each protocol graph structure, tracking modifications made during protocol execution, propagating approved modifications to the base version, and maintaining an audit trail of modifications including a healthcare provider associated with each of the modifications. In a fifth example of the method, optionally including one or more or each of the first through fourth examples, automatically updating the current position comprises: receiving real-time patient data from medical devices, caching the patient data in a local state manager, evaluating decision node criteria against cached patient data, identifying when patient data satisfies multiple decision criteria, and generating alerts when conflicting paths are identified. In a sixth example of the method, optionally including one or more or each of the first through fifth examples, the method further comprises: managing multiple concurrent protocol instances by: identifying related protocol graph structures based on shared patient parameters, synchronizing updates across related protocol instances, detecting conflicts between protocol requirements, and providing a resolution interface for healthcare providers to resolve identified conflicts.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative only and should not be construed to be limiting in any manner.

## Claims

1. A method (200) for digitizing medical protocols, comprising:
receiving an unstructured medical protocol (114);
converting the unstructured medical protocol (114) into machine-readable text;
analyzing the machine-readable text using natural language processing to:
identify action nodes representing medical actions;
identify decision nodes representing conditional branches; and
determine relationships between the action nodes and decision nodes;
generating a protocol graph structure (318) based on the identified nodes and relationships, wherein:
the protocol graph structure (318) comprises nodes and edges;
each node includes a position identifier and a parent position identifier; and
the edges represent transitions between nodes;
storing the protocol graph structure (318) in a database (112); and
generating a visual representation of the protocol graph structure (318) showing available paths through the medical protocol.

2. The method (200) of claim 1, wherein analyzing the machine-readable text comprises:
training a named entity recognition model using annotated medical protocols to identify:
medical actions comprising medication administration and patient monitoring tasks;
decision criteria comprising threshold values for patient vital signs; and
temporal relationships between actions and decisions; and
applying the trained named entity recognition model to the machine-readable text.

3. The method (200) of claim 1, wherein generating the protocol graph structure (318) comprises:
creating nodes comprising position identifiers and parent position identifiers;
establishing edges between nodes based on identified temporal and causal relationships;
validating the protocol graph structure (318) by:
verifying all decision nodes have corresponding action nodes;
checking for disconnected nodes; and
identifying circular references.

4. The method (200) of claim 1, further comprising:
receiving patient state data (120) from a hospital information system (116);
mapping the patient state data (120) to decision nodes in the protocol graph structure (318) using a medical terminology dictionary;
automatically updating a current position within the protocol graph structure (318) based on the mapped patient state data (120); and
generating alerts when the patient state data (120) triggers a transition between nodes.

5. The method (200) of claim 4, wherein mapping the patient state data (120) comprises:
maintaining a data mapping comprising:
canonical representations for clinical parameters;
standardized units for measurements;
pre-determined ranges for vital signs; and
hierarchical relationships between clinical concepts; and
converting received patient state data (120) into the canonical representations.

6. The method (200) of claim 1, further comprising:
creating an instance of the protocol graph structure (318) for a patient;
recording timestamps and healthcare provider notes for each traversed node;
maintaining an audit trail of override actions and manual position updates; and
archiving the instance upon reaching a terminal node.

7. The method (200) of claim 1, wherein generating the visual representation comprises:
displaying a current node in a first visual style;
displaying available next nodes based on current patient state data (120);
providing interactive elements for healthcare provider input at decision nodes; and
maintaining a visual history of traversed nodes.

8. A system (100) for digitizing medical protocols, comprising:
a computing device comprising:
one or more processors;
a display device (118); and
a memory storing instructions executable by the one or more processors to:
receive an unstructured medical protocol (114) via an input interface, wherein the unstructured medical protocol (114) comprises at least one of handwritten text, plain text, or spoken input;
convert the unstructured medical protocol (114) into machine-readable text;
analyze the machine-readable text using natural language processing to:
identify action nodes representing medical actions to be performed;
identify decision nodes representing patient state-dependent branches; and
determine relationships between the action nodes and decision nodes;
generate a protocol graph structure (318) comprising:
nodes representing the identified action nodes and decision nodes, wherein each node includes a position identifier and a parent position identifier; and
edges representing the determined relationships between nodes;
receive patient state data (120) from a hospital information system (116);
automatically update a current position within the protocol graph structure (318) based on the received patient state data (120); and
output, to the display device (118), a visual representation of the protocol graph structure (318) showing:
available paths through the medical protocol;
the current position within the protocol graph structure (318); and
historical progression through previously traversed nodes.

9. The system (100) of claim 8, wherein the instructions are further executable to:
create a protocol instance for a patient by:
associating the protocol graph structure (318) with a patient identifier;
initializing the current position within the protocol graph structure (318);
linking the protocol instance with patient data in the hospital information system (116); and
storing the protocol instance in a database (112).

10. The system (100) of claim 8, wherein the instructions are further executable to:
receive real-time patient data from medical devices connected to the hospital information system (116);
automatically map the real-time patient data to decision criteria in the protocol graph structure (318);
update the current position within the protocol graph structure (318) based on the mapped patient data; and
generate alerts when the updated position is associated with medical attention.

11. The system (100) of claim 8, wherein updating the current position comprises instructions executable to:
maintain a state cache of patient measurements;
evaluate decision node criteria against the state cache;
synchronize protocol instance states across distributed system (100) components; and
log state transitions with associated timestamps.

12. The system (100) of claim 8, wherein the instructions are further executable to:
manage multiple concurrent protocol instances for a single patient by:
storing, in a protocol instance database (112), multiple active protocol graph structures (318) associated with a patient identifier;
maintaining a mapping table defining clinical relationships between nodes of different protocol graph structures (318);
automatically updating patient positions within related protocol graph structures (318) when:
a measured patient parameter satisfies decision criteria in a first protocol graph structure (318), and
the measured patient parameter is mapped to decision criteria in at least one other protocol graph structure (318);
comparing medication dosages and treatment timing requirements between concurrent protocol graph structures (318);
identifying conflicts when:
two or more protocol graph structures (318) specify contradictory medications; or
timing requirements between protocol steps are incompatible; and generating, on the display device (118), an alert comprising:
identifiers of the two or more protocol graph structures (318);
specific nodes containing conflicting requirements; and
patient parameters related to the identified conflicts.

13. The system (100) of claim 8, wherein the instructions are further executable to:
provide an override interface enabling healthcare providers to:
manually override automated position updates;
document reasons for overrides;
specify alternative paths through the protocol graph structure (318); and
obtain authorization for overrides based on provider credentials.

14. The system (100) of claim 8, wherein the instructions are further executable to:
maintain version control of protocol graph structures (318) by:
storing a base version of each protocol graph structure (318);
tracking modifications made during protocol execution;
propagating approved modifications to the base version; and
maintaining an audit trail of modifications including a healthcare provider associated with each of the modifications.

15. The system (100) of claim 8, wherein the instructions are further executable to:
manage multiple concurrent protocol instances by:
identifying related protocol graph structures (318) based on shared patient parameters;
synchronizing updates across related protocol instances;
detecting conflicts between protocol requirements; and
providing a resolution interface for healthcare providers to resolve identified conflicts.
